# EUROPEAN PATENT SPECIFICATION

(11) **EP 3 062 809 B1**
(45) Date of publication and mention of the grant of the patent: **08.07.2020**
(21) Application number: 14802211.4
(22) Date of filing: 30.10.2014
(51) Int. Cl.: A61K 38/10, A61K 31/5377, A61K 31/7068, A61P 35/00

(54) **A CXCR4 PEPTIDE INHIBITOR FOR USE IN TREATING ACUTE MYELOID LEUKEMIA WITH A FLT3 MUTATION**
EIN CXCR4 PEPTIDE INHIBITOR ZUR BEHANDLUNG VON AKUTER MYELOISCHER LEUKÄMIE MIT EINER FLT3-MUTATION
UN INHIBITEUR PEPTIDIQUE DE CXCR4 POUR LE TRAITEMENT DE LA LEUCÉMIE MYÉLOÏDE AIGUË AVEC MUTATION DE LA FLT3

(30) Priority: 31.10.2013 US 201361897921 P; 27.02.2014 US 201461945302 P
(43) Date of publication of application: 07.09.2016
(73) Proprietor: Biokine Therapeutics Ltd., 7403617 Nes Ziona (IL); BioLineRx Ltd., 7177871 Modiin (IL)
(72) Inventor: PELED, Amnon, 6777634 Tel-Aviv (IL); ABRAHAM, Michal, 9077243 Mevasseret Zion (IL)
(74) Representative: Inspicos P/S
(86) International application number: PCT/IL2014/050939
(87) International publication number: WO 2015/063768

(56) References cited:
- ZENG ZHIHONG ET AL: "Inhibition of CXCR4 with the novel RCP168 peptide overcomes stroma-mediated chemoresistance in chronic and acute leukemias", MOLECULAR CANCER THERAPEUTICS, AMERICAN ASSOCIATION OF CANCER RESEARCH, US, vol. 5, no. 12, 1 December 2006 (2006-12-01), pages 3113-3121, XP008139367, ISSN: 1535-7163, DOI: 10.1158/1535-7163
- KATIA BEIDER ET AL: "CXCR4 antagonist 4F-benzoyl-TN14003 inhibits leukemia and multiple myeloma tumor growth", EXPERIMENTAL HEMATOLOGY, ELSEVIER INC, US, vol. 39, no. 3, 30 November 2010 (2010-11-30), pages 282-292, XP028161350, ISSN: 0301-472X, DOI: 10.1016/J.EXPHEM.2010.11.010 [retrieved on 2010-12-05]
- JACOBI A ET AL: "Impact of CXCR4 inhibition on FLT3-ITDpositive human AML blasts", EXPERIMENTAL HEMATOLOGY, ELSEVIER INC, US, vol. 38, no. 3, 1 March 2010 (2010-03-01), pages 180-190, XP026913582, ISSN: 0301-472X, DOI: 10.1016/J.EXPHEM.2009.12.003 [retrieved on 2010-02-18]
- ZENG Z ET AL: "Targeting the leukemia microenvironment by CXCR4 inhibition overcomes resistance to kinase inhibitors and chemotherapy in AML", BLOOD, AMERICAN SOCIETY OF HEMATOLOGY, US, vol. 113, no. 24, 11 June 2009 (2009-06-11), pages 6215-6224, XP002716127, ISSN: 0006-4971, DOI: 10.1182/BLOOD-2008-05-158311 [retrieved on 2008-10-27]
- QIN T ET AL: "Effect of Cytarabine and Decitabine in combination in human leukemic cell lines", CLINICAL CANCER RESEARCH, THE AMERICAN ASSOCIATION FOR CANCER RESEARCH, US, vol. 13, no. 14, 15 July 2007 (2007-07-15) , pages 4225-4232, XP002725241, ISSN: 1078-0432
- AMNON PELED ET AL: "Role of CXCR4 in the Pathogenesis of Acute Myeloid Leukemia", THERANOSTICS, vol. 3, no. 1, 1 January 2013 (2013-01-01) , pages 34-39, XP055164251, ISSN: 1838-7640, DOI: 10.7150/thno.5150
- Yaron Pereg ET AL: "BL-8040, a CXCR4 antagonist, synergizes with the FLT3 inhibitor AC220 inducing apoptosis and reducing minimal residual disease to prolong survival of AML diseased mice", SOHO Annual Meeting Proceedings, 17 September 2014 (2014-09-17), page 203, XP055164272, Retrieved from the Internet: URL:http://soho2014.com/wp-content/uploads /203.pdf [retrieved on 2015-01-22]

### SEQUENCE LISTING

<110> Biokine Therapeutics Ltd.
   BIOLINERX LTD.
   PELED, Amnon
   ABRAHAM, Michal
<120> METHODS OF TREATING ACUTE MYELOID LEUKEMIA WITH A FLT3 MUTATION
<130> 59549
<150> US 61/897,921
   <151> 2013-10-31
<150> US 61/945,302
   <151> 2014-02-27
<160> 74
<170> PatentIn version 3.5
<210> 1
   <211> 14
   <212> PRT
   <213> Artificial
<220>
   <223> synthetic peptide
<220>
   <221> MOD_RES
   <222> (1)..(1)
   <223> 4-fluorobenzoyl-arginine
<220>
   <221> MOD_RES
   <222> (3)..(3)
   <223> 3-((2-naphthyl) alanine
<220>
   <221> MOD_RES
   <222> (6)..(6)
   <223> citrulline
<220>
   <221> MOD_RES
   <222> (8)..(8)
   <223> D-lysine
<220>
   <221> MOD_RES
   <222> (12)..(12)
   <223> citrulline
<220>
   <221> MOD_RES
   <222> (14)..(14)
   <223> C' AMIDATED
<400> 1
   Xaa Arg Xaa Cys Tyr Xaa Lys Xaa Pro Tyr Arg Xaa Cys Arg
   1               5                   10
<210> 2
   <211> 14
   <212> PRT
   <213> Artificial
<220>
   <223> SYNTHETIC PEPTIDE
<220>
   <221> MOD_RES
   <222> (1)..(1)
   <223> N' ACETYLATION
<220>
   <221> MOD_RES
   <222> (3)..(3)
   <223> 3-((2-naphthyl) alanine
<220>
   <221> MOD_RES
   <222> (6)..(6)
   <223> citrulline
<220>
   <221> MOD_RES
   <222> (8)..(8)
   <223> D-lysine
<220>
   <221> MOD_RES
   <222> (12)..(12)
   <223> citrulline
<400> 2
   Arg Arg Xaa Cys Tyr Xaa Lys Xaa Pro Tyr Arg Xaa Cys Arg
   1               5                   10
<210> 3
   <211> 14
   <212> PRT
   <213> Artificial
<220>
   <223> SYNTHETIC PEPTIDE
<220>
   <221> MOD_RES
   <222> (1)..(1)
   <223> N' ACETYLATION
<220>
   <221> MOD_RES
   <222> (3)..(3)
   <223> 3-((2-naphthyl) alanine
<220>
   <221> MOD_RES
   <222> (8)..(8)
   <223> D-citrulline
<220>
   <221> MOD_RES
   <222> (12)..(12)
   <223> citrulline
<400> 3
   Arg Arg Xaa Cys Tyr Arg Lys Xaa Pro Tyr Arg Xaa Cys Arg
   1               5                   10
<210> 4
   <211> 14
   <212> PRT
   <213> Artificial
<220>
   <223> synthetic peptide
<220>
   <221> MOD_RES
   <222> (1)..(1)
   <223> N' ACETYLATED
<220>
   <221> MOD_RES
   <222> (3)..(3)
   <223> 3-((2-naphthyl) alanine
<220>
   <221> MOD_RES
   <222> (6)..(6)
   <223> citrulline
<220>
   <221> MOD_RES
   <222> (8)..(8)
   <223> D-citrulline
<220>
   <221> MOD_RES
   <222> (12)..(12)
   <223> citrulline
<400> 4
   Arg Arg Xaa Cys Tyr Xaa Lys Xaa Pro Tyr Arg Xaa Cys Arg
   1               5                   10
<210> 5
   <211> 14
   <212> PRT
   <213> Artificial
<220>
   <223> synthetic peptide
<220>
   <221> MOD_RES
   <222> (1)..(1)
   <223> N' ACETYLATED
<220>
   <221> MOD_RES
   <222> (3)..(3)
   <223> 3-((2-naphthyl) alanine
<220>
   <221> MOD_RES
   <222> (6)..(6)
   <223> citrulline
<220>
   <221> MOD_RES
   <222> (8)..(8)
   <223> D-lysine
<220>
   <221> MOD_RES
   <222> (11)..(12)
   <223> citrulline
<400> 5
   Arg Arg Xaa Cys Tyr Xaa Lys Xaa Pro Tyr Xaa Xaa Cys Arg
   1               5                   10
<210> 6
   <211> 14
   <212> PRT
   <213> Artificial
<220>
   <223> synthetic peptide
<220>
   <221> MOD_RES
   <222> (1)..(1)
   <223> N' acetylated citrulline
<220>
   <221> MOD_RES
   <222> (3)..(3)
   <223> 3-((2-naphthyl) alanine
<220>
   <221> MOD_RES
   <222> (6)..(6)
   <223> citrulline
<220>
   <221> MOD_RES
   <222> (8)..(8)
   <223> D-lysine
<220>
   <221> MOD_RES
   <222> (12).. (12)
   <223> citrulline
<400> 6
   Xaa Arg Xaa Cys Tyr Xaa Lys Xaa Pro Tyr Arg Xaa Cys Arg
   1               5                   10
<210> 7
   <211> 14
   <212> PRT
   <213> Artificial
<220>
   <223> synthetic peptide
<220>
   <221> MOD_RES
   <222> (1)..(1)
   <223> N' acetylated citrulline
<220>
   <221> MOD_RES
   <222> (3)..(3)
   <223> 3-((2-naphthyl) alanine
<220>
   <221> MOD_RES
   <222> (8)..(8)
   <223> D-citrulline
<220>
   <221> MOD_RES
   <222> (12)..(12)
   <223> citrulline
<400> 7
   Xaa Arg Xaa Cys Tyr Arg Lys Xaa Pro Tyr Arg Xaa Cys Arg
   1               5                   10
<210> 8
   <211> 14
   <212> PRT
   <213> Artificial
<220>
   <223> synthetic peptide
<220>
   <221> MOD_RES
   <222> (1)..(1)
   <223> N' ACETYLATED
<220>
   <221> MOD_RES
   <222> (3)..(3)
   <223> 3-((2-naphthyl) alanine
<220>
   <221> MOD_RES
   <222> (8)..(8)
   <223> D-citrulline
<220>
   <221> MOD_RES
   <222> (11)..(12)
   <223> citrulline
<400> 8
   Arg Arg Xaa Cys Tyr Arg Lys Xaa Pro Tyr Xaa Xaa Cys Arg
   1 5 10
<210> 9
   <211> 14
   <212> PRT
   <213> Artificial
<220>
   <223> synthetic peptide
<220>
   <221> MOD_RES
   <222> (1)..(1)
   <223> N' ACETYLATED citrulline
<220>
   <221> MOD_RES
   <222> (3)..(3)
   <223> 3-((2-naphthyl) alanine
<220>
   <221> MOD_RES
   <222> (8)..(8)
   <223> D-lysine
<220>
   <221> MOD_RES
   <222> (11)..(12)
   <223> citrulline
<400> 9
   Xaa Arg Xaa Cys Tyr Arg Lys Xaa Pro Tyr Xaa Xaa Cys Arg
   1 5 10
<210> 10
   <211> 14
   <212> PRT
   <213> Artificial
<220>
   <223> synthetic peptide
<220>
   <221> MOD_RES
   <222> (1)..(1)
   <223> N' ACETYLATED
<220>
   <221> MOD_RES
   <222> (3)..(3)
   <223> 3-((2-naphthyl) alanine
<220>
   <221> MOD_RES
   <222> (6)..(6)
   <223> citrulline
<220>
   <221> MOD_RES
   <222> (8)..(8)
   <223> D-citrulline
<220>
   <221> MOD_RES
   <222> (12)..(12)
   <223> citrulline
<220>
   <221> MOD_RES
   <222> (14)..(14)
   <223> C' AMIDATED
<400> 10
   Arg Arg Xaa Cys Tyr Xaa Lys Xaa Pro Tyr Arg Xaa Cys Arg
   1               5                   10
<210> 11
   <211> 14
   <212> PRT
   <213> Artificial
<220>
   <223> synthetic peptide
<220>
   <221> MOD_RES
   <222> (1)..(1)
   <223> N' ACETYLATED
<220>
   <221> MOD_RES
   <222> (3)..(3)
   <223> 3-((2-naphthyl) alanine
<220>
   <221> MOD_RES
   <222> (6).. (6)
   <223> citrulline
<220>
   <221> MOD_RES
   <222> (8)..(8)
   <223> D-lysine
<220>
   <221> MOD_RES
   <222> (11)..(12)
   <223> citrulline
<220>
   <221> MOD_RES
   <222> (14)..(14)
   <223> C' AMIDATED
<400> 11
   Arg Arg Xaa Cys Tyr Xaa Lys Xaa Pro Tyr Xaa Xaa Cys Arg
   1               5                   10
<210> 12
   <211> 14
   <212> PRT
   <213> Artificial
<220>
   <223> synthetic peptide
<220>
   <221> MOD_RES
   <222> (1)..(1)
   <223> N' ACETYLATED citrulline
<220>
   <221> MOD_RES
   <222> (3)..(3)
   <223> 3-((2-naphthyl) alanine
<220>
   <221> MOD_RES
   <222> (6)..(6)
   <223> citrulline
<220>
   <221> MOD_RES
   <222> (8)..(8)
   <223> D-lysine
<220>
   <221> MOD_RES
   <222> (12)..(12)
   <223> citrulline
<220>
   <221> MOD_RES
   <222> (14)..(14)
   <223> AMIDATED
<400> 12
   Xaa Arg Xaa Cys Tyr Xaa Lys Xaa Pro Tyr Arg Xaa Cys Arg
   1               5                   10
<210> 13
   <211> 14
   <212> PRT
   <213> Artificial
<220>
   <223> synthetic peptide
<220>
   <221> MOD_RES
   <222> (1)..(1)
   <223> N' ACETYLATED citrulline
<220>
   <221> MOD_RES
   <222> (3)..(3)
   <223> 3-((2-naphthyl) alanine
<220>
   <221> MOD_RES
   <222> (8)..(8)
   <223> D-citrulline
<220>
   <221> MOD_RES
   <222> (12)..(12)
   <223> citrulline
<220>
   <221> MOD_RES
   <222> (14)..(14)
   <223> C' AMIDATED
<400> 13
   Xaa Arg Xaa Cys Tyr Arg Lys Xaa Pro Tyr Arg Xaa Cys Arg
   1               5                   10
<210> 14
   <211> 14
   <212> PRT
   <213> Artificial
<220>
   <223> synthetic peptide
<220>
   <221> MOD_RES
   <222> (1)..(1)
   <223> N' ACETYLATED
<220>
   <221> MOD_RES
   <222> (3)..(3)
   <223> 3-((2-naphthyl) alanine
<220>
   <221> MOD_RES
   <222> (8)..(8)
   <223> D-citrulline
<220>
   <221> MOD_RES
   <222> (11)..(12)
   <223> citrulline
<220>
   <221> MOD_RES
   <222> (14)..(14)
   <223> C' AMIDATED
<400> 14
   Arg Arg Xaa Cys Tyr Arg Lys Xaa Pro Tyr Xaa Xaa Cys Arg
   1               5                   10
<210> 15
   <211> 14
   <212> PRT
   <213> Artificial
<220>
   <223> synthetic peptide
<220>
   <221> MOD_RES
   <222> (1)..(1)
   <223> N' ACETYLATED citrulline
<220>
   <221> MOD_RES
   <222> (3)..(3)
   <223> 3-((2-naphthyl) alanine
<220>
   <221> MOD_RES
   <222> (8)..(8)
   <223> D-lysine
<220>
   <221> MOD_RES
   <222> (11)..(12)
   <223> citrulline
<220>
   <221> MOD_RES
   <222> (14)..(14)
   <223> C' AMIDATED
<400> 15
   Xaa Arg Xaa Cys Tyr Arg Lys Xaa Pro Tyr Xaa Xaa Cys Arg
   1               5                   10
<210> 16
   <211> 14
   <212> PRT
   <213> Artificial
<220>
   <223> synthetic peptide
<220>
   <221> MOD_RES
   <222> (1)..(1)
   <223> D-glutamic acid
<220>
   <221> MOD_RES
   <222> (3)..(3)
   <223> 3-((2-naphthyl) alanine
<220>
   <221> MOD_RES
   <222> (8)..(8)
   <223> D-lysine
<220>
   <221> MOD_RES
   <222> (12)..(12)
   <223> citrulline
<400> 16
   Xaa Arg Xaa Cys Tyr Arg Lys Xaa Pro Tyr Arg Xaa Cys Arg
   1               5                   10
<210> 17
   <211> 14
   <212> PRT
   <213> Artificial
<220>
   <223> synthetic peptide
<220>
   <221> MOD_RES
   <222> (3)..(3)
   <223> 3-((2-naphthyl) alanine
<220>
   <221> MOD_RES
   <222> (8)..(8)
   <223> D-lysine
<220>
   <221> MOD_RES
   <222> (12)..(12)
   <223> citrulline
<400> 17
   Arg Glu Xaa Cys Tyr Arg Lys Xaa Pro Tyr Arg Xaa Cys Arg
   1               5                   10
<210> 18
   <211> 14
   <212> PRT
   <213> Artificial
<220>
   <223> synthetic peptide
<220>
   <221> MOD_RES
   <222> (3)..(3)
   <223> 3-((2-naphthyl) alanine
<220>
   <221> MOD_RES
   <222> (8)..(8)
   <223> D-lysine
<220>
   <221> MOD_RES
   <222> (12)..(12)
   <223> citrulline
<400> 18
   Arg Arg Xaa Cys Tyr Glu Lys Xaa Pro Tyr Arg Xaa Cys Arg
   1 5 10
<210> 19
   <211> 14
   <212> PRT
   <213> Artificial
<220>
   <223> synthetic peptide
<220>
   <221> MOD_RES
   <222> (3).. (3)
   <223> 3-((2-naphthyl) alanine
<220>
   <221> MOD_RES
   <222> (8)..(8)
   <223> D-lysine
<220>
   <221> MOD_RES
   <222> (12)..(12)
   <223> citrulline
<400> 19
   Arg Arg Xaa Cys Tyr Arg Glu Xaa Pro Tyr Arg Xaa Cys Arg
   1 5 10
<210> 20
   <211> 14
   <212> PRT
   <213> Artificial
<220>
   <223> synthetic peptide
<220>
   <221> MOD_RES
   <222> (3)..(3)
   <223> 3-((2-naphthyl) alanine
<220>
   <221> MOD_RES
   <222> (8)..(8)
   <223> D-glutamic acid
<220>
   <221> MOD_RES
   <222> (12)..(12)
   <223> citrulline
<400> 20
   Arg Arg Xaa Cys Tyr Arg Lys Xaa Pro Tyr Arg Xaa Cys Arg
   1               5                   10
<210> 21
   <211> 14
   <212> PRT
   <213> Artificial
<220>
   <223> synthetic peptide
<220>
   <221> MOD_RES
   <222> (3)..(3)
   <223> 3-((2-naphthyl) alanine
<220>
   <221> MOD_RES
   <222> (8)..(8)
   <223> D-lysine
<220>
   <221> MOD_RES
   <222> (12)..(12)
   <223> citrulline
<400> 21
   Arg Arg Xaa Cys Tyr Arg Lys Xaa Pro Tyr Glu Xaa Cys Arg
   1               5                   10
<210> 22
   <211> 14
   <212> PRT
   <213> Artificial
<220>
   <223> synthetic peptide
<220>
   <221> MOD_RES
   <222> (3)..(3)
   <223> 3-((2-naphthyl) alanine
<220>
   <221> MOD_RES
   <222> (8)..(8)
   <223> D-lysine
<220>
   <221> MOD_RES
   <222> (12)..(12)
   <223> citrulline
<400> 22
   Arg Arg Xaa Cys Tyr Arg Lys Xaa Pro Tyr Arg Xaa Cys Glu
   1               5                   10
<210> 23
   <211> 14
   <212> PRT
   <213> Artificial
<220>
   <223> synthetic peptide
<220>
   <221> MOD_RES
   <222> (3)..(3)
   <223> 3-((2-naphthyl) alanine
<220>
   <221> MOD_RES
   <222> (6)..(6)
   <223> citrulline
<220>
   <221> MOD_RES
   <222> (8)..(8)
   <223> D-glutamic acid
<220>
   <221> MOD_RES
   <222> (12)..(12)
   <223> citrulline
<220>
   <221> MOD_RES
   <222> (14)..(14)
   <223> C' amidated
<400> 23
   Arg Arg Xaa Cys Tyr Xaa Lys Xaa Pro Tyr Arg Xaa Cys Arg
   1 5 10
<210> 24
   <211> 14
   <212> PRT
   <213> Artificial
<220>
   <223> synthetic peptide
<220>
   <221> MOD_RES
   <222> (3)..(3)
   <223> 3-((2-naphthyl) alanine
<220>
   <221> MOD_RES
   <222> (6)..(6)
   <223> D-glutamic acid
<220>
   <221> MOD_RES
   <222> (8)..(8)
   <223> D-citrulline
<220>
   <221> MOD_RES
   <222> (12)..(12)
   <223> citrulline
<220>
   <221> MOD_RES
   <222> (14)..(14)
   <223> c' amidated
<400> 24
   Arg Arg Xaa Cys Tyr Xaa Lys Xaa Pro Tyr Arg Xaa Cys Arg
   1 5 10
<210> 25
   <211> 14
   <212> PRT
   <213> Artificial
<220>
   <223> synthetic peptide
<220>
   <221> MOD_RES
   <222> (3)..(3)
   <223> 3-((2-naphthyl) alanine
<220>
   <221> MOD_RES
   <222> (6)..(6)
   <223> D-glutamic acid
<220>
   <221> MOD_RES
   <222> (8)..(8)
   <223> D-glutamic acid
<220>
   <221> MOD_RES
   <222> (12)..(12)
   <223> citrulline
<220>
   <221> MOD_RES
   <222> (14)..(14)
   <223> c' amidated
<400> 25
   Arg Arg Xaa Cys Tyr Xaa Lys Xaa Pro Tyr Arg Xaa Cys Arg
   1               5                   10
<210> 26
   <211> 14
   <212> PRT
   <213> Artificial
<220>
   <223> synthetic peptide
<220>
   <221> MOD_RES
   <222> (1)..(1)
   <223> D-glutamic acid
<220>
   <221> MOD_RES
   <222> (3)..(3)
   <223> 3-((2-naphthyl) alanine
<220>
   <221> MOD_RES
   <222> (6)..(6)
   <223> citrulline
<220>
   <221> MOD_RES
   <222> (8)..(8)
   <223> D-glutamic acid
<220>
   <221> MOD_RES
   <222> (12)..(12)
   <223> citrulline
<220>
   <221> MOD_RES
   <222> (14)..(14)
   <223> c' amidated
<400> 26
   Xaa Arg Xaa Cys Tyr Xaa Lys Xaa Pro Tyr Arg Xaa Cys Arg
   1 5 10
<210> 27
   <211> 14
   <212> PRT
   <213> Artificial
<220>
   <223> synthetic peptide
<220>
   <221> MOD_RES
   <222> (3)..(3)
   <223> 3-((2-naphthyl) alanine
<220>
   <221> MOD_RES
   <222> (6)..(6)
   <223> citrulline
<220>
   <221> MOD_RES
   <222> (8)..(8)
   <223> D-glutamic acid
<220>
   <221> MOD_RES
   <222> (10)..(10)
   <223> D-glutamic acid
<220>
   <221> MOD_RES
   <222> (12)..(12)
   <223> citrulline
<220>
   <221> MOD_RES
   <222> (14)..(14)
   <223> c' amidated
<400> 27
   Arg Arg Xaa Cys Tyr Xaa Lys Xaa Pro Xaa Arg Xaa Cys Arg
   1 5 10
<210> 28
   <211> 14
   <212> PRT
   <213> Artificial
<220>
   <223> synthetic peptide
<220>
   <221> MOD_RES
   <222> (3)..(3)
   <223> 3-((2-naphthyl) alanine
<220>
   <221> MOD_RES
   <222> (6).. (6)
   <223> citrulline
<220>
   <221> MOD_RES
   <222> (8)..(8)
   <223> D-glutamic acid
<220>
   <221> MOD_RES
   <222> (12)..(12)
   <223> D-glutamic acid
<220>
   <221> MOD_RES
   <222> (14)..(14)
   <223> c' amidated
<400> 28
   Arg Arg Xaa Cys Tyr Xaa Lys Xaa Pro Tyr Arg Xaa Cys Arg
   1               5                   10
<210> 29
   <211> 14
   <212> PRT
   <213> Artificial
<220>
   <223> synthetic peptide
<220>
   <221> MOD_RES
   <222> (1)..(1)
   <223> N' ACETYLATED D-glutamic acid
<220>
   <221> MOD_RES
   <222> (3)..(3)
   <223> 3-((2-naphthyl) alanine
<220>
   <221> MOD_RES
   <222> (6)..(6)
   <223> citrulline
<220>
   <221> MOD_RES
   <222> (8)..(8)
   <223> D-glutamic acid
<220>
   <221> MOD_RES
   <222> (12)..(12)
   <223> citrulline
<220>
   <221> MOD_RES
   <222> (14)..(14)
   <223> c' amidated
<400> 29
   Xaa Arg Xaa Cys Tyr Xaa Lys Xaa Pro Tyr Arg Xaa Cys Arg
   1               5                   10
<210> 30
   <211> 14
   <212> PRT
   <213> Artificial
<220>
   <223> synthetic peptide
<220>
   <221> MOD_RES
   <222> (1)..(1)
   <223> N' ACETYLATED
<220>
   <221> MOD_RES
   <222> (3)..(3)
   <223> 3-((2-naphthyl) alanine
<220>
   <221> MOD_RES
   <222> (6)..(6)
   <223> citrulline
<220>
   <221> MOD_RES
   <222> (8)..(8)
   <223> D-glutamic acid
<220>
   <221> MOD_RES
   <222> (10)..(10)
   <223> D-glutamic acid
<220>
   <221> MOD_RES
   <222> (12)..(12)
   <223> citrulline
<220>
   <221> MOD_RES
   <222> (14)..(14)
   <223> c' amidated
<400> 30
   Arg Arg Xaa Cys Tyr Xaa Lys Xaa Pro Xaa Arg Xaa Cys Arg
   1 5 10
<210> 31
   <211> 14
   <212> PRT
   <213> Artificial
<220>
   <223> synthetic peptide
<220>
   <221> MOD_RES
   <222> (1)..(1)
   <223> N' ACETYLATED
<220>
   <221> MOD_RES
   <222> (3)..(3)
   <223> 3-((2-naphthyl) alanine
<220>
   <221> MOD_RES
   <222> (6)..(6)
   <223> citrulline
<220>
   <221> MOD_RES
   <222> (8)..(8)
   <223> D-glutamic acid
<220>
   <221> MOD_RES
   <222> (12)..(12)
   <223> D-glutamic acid
<220>
   <221> MOD_RES
   <222> (14)..(14)
   <223> c' amidated
<400> 31
   Arg Arg Xaa Cys Tyr Xaa Lys Xaa Pro Tyr Arg Xaa Cys Arg
   1 5 10
<210> 32
   <211> 14
   <212> PRT
   <213> Artificial
<220>
   <223> synthetic peptide
<220>
   <221> MOD_RES
   <222> (1)..(1)
   <223> N' ACETYLATED
<220>
   <221> MOD_RES
   <222> (3)..(3)
   <223> 3-((2-naphthyl) alanine
<220>
   <221> MOD_RES
   <222> (6)..(6)
   <223> citrulline
<220>
   <221> MOD_RES
   <222> (8)..(8)
   <223> D-glutamic acid
<220>
   <221> MOD_RES
   <222> (12)..(12)
   <223> citrulline
<220>
   <221> MOD_RES
   <222> (14)..(14)
   <223> c' amidated
<400> 32
   Arg Arg Xaa Cys Tyr Xaa Lys Xaa Pro Tyr Arg Xaa Cys Arg
   1               5                   10
<210> 33
   <211> 14
   <212> PRT
   <213> Artificial
<220>
   <223> synthetic peptide
<220>
   <221> MOD_RES
   <222> (1)..(1)
   <223> Guanyl-arginine
<220>
   <221> MOD_RES
   <222> (3)..(3)
   <223> 3-((2-naphthyl) alanine
<220>
   <221> MOD_RES
   <222> (6)..(6)
   <223> citrulline
<220>
   <221> MOD_RES
   <222> (8)..(8)
   <223> D-glutamic acid
<220>
   <221> MOD_RES
   <222> (12)..(12)
   <223> citrulline
<220>
   <221> MOD_RES
   <222> (14)..(14)
   <223> c' amidated
<400> 33
   Xaa Arg Xaa Cys Tyr Xaa Lys Xaa Pro Tyr Arg Xaa Cys Arg
   1               5                   10
<210> 34
   <211> 14
   <212> PRT
   <213> Artificial
<220>
   <223> synthetic peptide
<220>
   <221> MOD_RES
   <222> (1)..(1)
   <223> Tetramethylguanyl-arginine
<220>
   <221> MOD_RES
   <222> (3)..(3)
   <223> 3-((2-naphthyl) alanine
<220>
   <221> MOD_RES
   <222> (6)..(6)
   <223> citrulline
<220>
   <221> MOD_RES
   <222> (8)..(8)
   <223> D-glutamic acid
<220>
   <221> MOD_RES
   <222> (12)..(12)
   <223> citrulline
<220>
   <221> MOD_RES
   <222> (14)..(14)
   <223> c' amidated
<400> 34
   Xaa Arg Xaa Cys Tyr Xaa Lys Xaa Pro Tyr Arg Xaa Cys Arg
   1               5                   10
<210> 35
   <211> 13
   <212> PRT
   <213> Artificial
<220>
   <223> synthetic peptide
<220>
   <221> MOD_RES
   <222> (1)..(1)
   <223> Tetramethylguanyl-arginine
<220>
   <221> MOD_RES
   <222> (2)..(2)
   <223> 3-((2-naphthyl) alanine
<220>
   <221> MOD_RES
   <222> (5)..(5)
   <223> citrulline
<220>
   <221> MOD_RES
   <222> (7).. (7)
   <223> D-glutamic acid
<220>
   <221> MOD_RES
   <222> (11)..(11)
   <223> citrulline
<220>
   <221> MOD_RES
   <222> (13)..(13)
   <223> c' amidated
<400> 35
   Xaa Xaa Cys Tyr Xaa Lys Xaa Pro Tyr Arg Xaa Cys Arg
   1               5                   10
<210> 36
   <211> 14
   <212> PRT
   <213> Artificial
<220>
   <223> synthetic peptide
<220>
   <221> MOD_RES
   <222> (1)..(1)
   <223> 4-fluorobenzoyl-arginine
<220>
   <221> MOD_RES
   <222> (3)..(3)
   <223> 3-((2-naphthyl) alanine
<220>
   <221> MOD_RES
   <222> (6)..(6)
   <223> citrulline
<220>
   <221> MOD_RES
   <222> (8)..(8)
   <223> D-glutamic acid
<220>
   <221> MOD_RES
   <222> (12)..(12)
   <223> citrulline
<220>
   <221> MOD_RES
   <222> (14)..(14)
   <223> amidated
<400> 36
   Xaa Arg Xaa Cys Tyr Xaa Lys Xaa Pro Tyr Arg Xaa Cys Arg
   1               5                   10
<210> 37
   <211> 14
   <212> PRT
   <213> Artificial
<220>
   <223> synthetic peptide
<220>
   <221> MOD_RES
   <222> (1)..(1)
   <223> 2-fluorobenzoyl-arginine
<220>
   <221> MOD_RES
   <222> (3)..(3)
   <223> 3-((2-naphthyl) alanine
<220>
   <221> MOD_RES
   <222> (6)..(6)
   <223> citrulline
<220>
   <221> MOD_RES
   <222> (8)..(8)
   <223> D-glutamic acid
<220>
   <221> MOD_RES
   <222> (12)..(12)
   <223> citrulline
<220>
   <221> MOD_RES
   <222> (14)..(14)
   <223> c' amidated
<400> 37
   Xaa Arg Xaa Cys Tyr Xaa Lys Xaa Pro Tyr Arg Xaa Cys Arg
   1               5                   10
<210> 38
   <211> 13
   <212> PRT
   <213> Artificial
<220>
   <223> synthetic peptide
<220>
   <221> MOD_RES
   <222> (1)..(1)
   <223> 5-aminopentanoyl-arginine
<220>
   <221> MOD_RES
   <222> (2) .. (2)
   <223> 3-((2-naphthyl) alanine
<220>
   <221> MOD_RES
   <222> (5)..(5)
   <223> citrulline
<220>
   <221> MOD_RES
   <222> (7).. (7)
   <223> D-glutamic acid
<220>
   <221> MOD_RES
   <222> (11)..(11)
   <223> citrulline
<220>
   <221> MOD_RES
   <222> (13)..(13)
   <223> c' amidated
<400> 38
   Xaa Xaa Cys Tyr Xaa Lys Xaa Pro Tyr Arg Xaa Cys Arg
   1               5                   10
<210> 39
   <211> 14
   <212> PRT
   <213> Artificial
<220>
   <223> synthetic peptide
<220>
   <221> MOD_RES
   <222> (1)..(1)
   <223> 2-desamino-arginyl
<220>
   <221> MOD_RES
   <222> (3)..(3)
   <223> 3-((2-naphthyl) alanine
<220>
   <221> MOD_RES
   <222> (6)..(6)
   <223> citrulline
<220>
   <221> MOD_RES
   <222> (8)..(8)
   <223> D-glutamic acid
<220>
   <221> MOD_RES
   <222> (12)..(12)
   <223> citrulline
<220>
   <221> MOD_RES
   <222> (14)..(14)
   <223> c' amidated
<400> 39
   Xaa Arg Xaa Cys Tyr Xaa Lys Xaa Pro Tyr Arg Xaa Cys Arg
   1               5                   10
<210> 40
   <211> 13
   <212> PRT
   <213> Artificial
<220>
   <223> synthetic peptide
<220>
   <221> MOD_RES
   <222> (1)..(1)
   <223> Guanyl-arginine
<220>
   <221> MOD_RES
   <222> (2).. (2)
   <223> 3-((2-naphthyl) alanine
<220>
   <221> MOD_RES
   <222> (5)..(5)
   <223> citrulline
<220>
   <221> MOD_RES
   <222> (7).. (7)
   <223> D-glutamic acid
<220>
   <221> MOD_RES
   <222> (11)..(11)
   <223> citrulline
<220>
   <221> MOD_RES
   <222> (13)..(13)
   <223> c' amidated
<400> 40
   Xaa Xaa Cys Tyr Xaa Lys Xaa Pro Tyr Arg Xaa Cys Arg
   1               5                   10
<210> 41
   <211> 13
   <212> PRT
   <213> Artificial
<220>
   <223> synthetic peptide
<220>
   <221> MOD_RES
   <222> (1)..(1)
   <223> Succinyl-arginine
<220>
   <221> MOD_RES
   <222> (2).. (2)
   <223> 3-((2-naphthyl) alanine
<220>
   <221> MOD_RES
   <222> (5)..(5)
   <223> citrulline
<220>
   <221> MOD_RES
   <222> (7).. (7)
   <223> D-glutamic acid
<220>
   <221> MOD_RES
   <222> (11)..(11)
   <223> citrulline
<220>
   <221> MOD_RES
   <222> (13)..(13)
   <223> c' amidated
<400> 41
   Xaa Xaa Cys Tyr Xaa Lys Xaa Pro Tyr Arg Xaa Cys Arg
   1               5                   10
<210> 42
   <211> 13
   <212> PRT
   <213> Artificial
<220>
   <223> synthetic peptide
<220>
   <221> MOD_RES
   <222> (1)..(1)
   <223> Glutaryl-arginine
<220>
   <221> MOD_RES
   <222> (2).. (2)
   <223> 3-((2-naphthyl) alanine
<220>
   <221> MOD_RES
   <222> (5)..(5)
   <223> citrulline
<220>
   <221> MOD_RES
   <222> (7)..(7)
   <223> D-glutamic acid
<220>
   <221> MOD_RES
   <222> (11)..(11)
   <223> citrulline
<220>
   <221> MOD_RES
   <222> (13)..(13)
   <223> c' amidated
<400> 42
   Xaa Xaa Cys Tyr Xaa Lys Xaa Pro Tyr Arg Xaa Cys Arg
   1 5 10
<210> 43
   <211> 14
   <212> PRT
   <213> Artificial
<220>
   <223> synthetic peptide
<220>
   <221> MISC_FEATURE
   <222> (1)..(1)
   <223> desaminoTMG-APA (formula IV in the specification)
<220>
   <221> MOD_RES
   <222> (3)..(3)
   <223> 3-((2-naphthyl) alanine
<220>
   <221> MOD_RES
   <222> (6)..(6)
   <223> citrulline
<220>
   <221> MOD_RES
   <222> (8)..(8)
   <223> D-glutamic acid
<220>
   <221> MOD_RES
   <222> (12)..(12)
   <223> citrulline
<220>
   <221> MOD_RES
   <222> (14)..(14)
   <223> c' amidated
<400> 43
   Xaa Arg Xaa Cys Tyr Xaa Lys Xaa Pro Tyr Arg Xaa Cys Arg
   1 5 10
<210> 44
   <211> 14
   <212> PRT
   <213> Artificial
<220>
   <223> synthetic peptide
<220>
   <221> MISC_FEATURE
   <222> (1)..(1)
   <223> R-CH2 - formula (V) in the specification
<220>
   <221> MOD_RES
   <222> (3)..(3)
   <223> 3-((2-naphthyl) alanine
<220>
   <221> MOD_RES
   <222> (6)..(6)
   <223> citrulline
<220>
   <221> MOD_RES
   <222> (8)..(8)
   <223> D-glutamic acid
<220>
   <221> MOD_RES
   <222> (12)..(12)
   <223> citrulline
<220>
   <221> MOD_RES
   <222> (14)..(14)
   <223> c' amidated
<400> 44
   Xaa Arg Xaa Cys Tyr Xaa Lys Xaa Pro Tyr Arg Xaa Cys Arg
   1               5                   10
<210> 45
   <211> 13
   <212> PRT
   <213> Artificial
<220>
   <223> synthetic peptide
<220>
   <221> MOD_RES
   <222> (2).. (2)
   <223> 3-((2-naphthyl) alanine
<220>
   <221> MOD_RES
   <222> (5)..(5)
   <223> citrulline
<220>
   <221> MOD_RES
   <222> (7)..(7)
   <223> D-glutamic acid
<220>
   <221> MOD_RES
   <222> (11)..(11)
   <223> citrulline
<220>
   <221> MOD_RES
   <222> (13)..(13)
   <223> c' amidated
<400> 45
   Arg Xaa Cys Tyr Xaa Lys Xaa Pro Tyr Arg Xaa Cys Arg
   1               5                   10
<210> 46
   <211> 14
   <212> PRT
   <213> Artificial
<220>
   <223> synthetic peptide
<220>
   <221> MOD_RES
   <222> (1)..(1)
   <223> tetramethylguanyl-arginine
<220>
   <221> MOD_RES
   <222> (3)..(3)
   <223> 3-((2-naphthyl)alanine
<220>
   <221> MOD_RES
   <222> (6)..(6)
   <223> citrulline
<220>
   <221> MOD_RES
   <222> (8)..(8)
   <223> D-citrulline
<220>
   <221> MOD_RES
   <222> (12)..(12)
   <223> citrulline
<220>
   <221> MOD_RES
   <222> (14)..(14)
   <223> C' AMIDATED
<400> 46
   Xaa Arg Xaa Cys Tyr Xaa Lys Xaa Pro Tyr Arg Xaa Cys Arg
   1               5                   10
<210> 47
   <211> 14
   <212> PRT
   <213> Artificial
<220>
   <223> synthetic peptide
<220>
   <221> MOD_RES
   <222> (1)..(1)
   <223> 6-aminohexanoyl-arginine
<220>
   <221> MOD_RES
   <222> (3)..(3)
   <223> 3-((2-naphthyl) alanine
<220>
   <221> MOD_RES
   <222> (6)..(6)
   <223> citrulline
<220>
   <221> MOD_RES
   <222> (8)..(8)
   <223> D-citrulline
<220>
   <221> MOD_RES
   <222> (12)..(12)
   <223> citrulline
<220>
   <221> MOD_RES
   <222> (14)..(14)
   <223> C' AMIDATED
<400> 47
   Xaa Arg Xaa Cys Tyr Xaa Lys Xaa Pro Tyr Arg Xaa Cys Arg
   1               5                   10
<210> 48
   <211> 14
   <212> PRT
   <213> Artificial
<220>
   <223> synthetic peptide
<220>
   <221> MOD_RES
   <222> (1)..(1)
   <223> 6-aminohexanoyl-arginine
<220>
   <221> MOD_RES
   <222> (3)..(3)
   <223> 3-((2-naphthyl) alanine
<220>
   <221> MOD_RES
   <222> (8)..(8)
   <223> D-lysine
<220>
   <221> MOD_RES
   <222> (12)..(12)
   <223> citrulline
<400> 48
   Xaa Arg Xaa Cys Tyr Arg Lys Xaa Pro Tyr Arg Xaa Cys Arg
   1               5                   10
<210> 49
   <211> 14
   <212> PRT
   <213> Artificial
<220>
   <223> synthetic peptide
<220>
   <221> MOD_RES
   <222> (3)..(3)
   <223> 3-((2-naphthyl) alanine
<220>
   <221> MOD_RES
   <222> (6)..(6)
   <223> citrulline
<220>
   <221> MOD_RES
   <222> (8)..(8)
   <223> D-lysine
<220>
   <221> MOD_RES
   <222> (12)..(12)
   <223> citrulline
<220>
   <221> MOD_RES
   <222> (14)..(14)
   <223> C' amidated
<400> 49
   Arg Arg Xaa Cys Tyr Xaa Arg Xaa Pro Tyr Arg Xaa Cys Arg
   1               5                   10
<210> 50
   <211> 14
   <212> PRT
   <213> Artificial
<220>
   <223> synthetic peptide
<220>
   <221> MOD_RES
   <222> (1)..(1)
   <223> N' ACETYLATED
<220>
   <221> MOD_RES
   <222> (3)..(3)
   <223> 3-((2-naphthyl) alanine
<220>
   <221> MOD_RES
   <222> (6)..(6)
   <223> citrulline
<220>
   <221> MOD_RES
   <222> (8)..(8)
   <223> D-lysine
<220>
   <221> MOD_RES
   <222> (12)..(12)
   <223> citrulline
<220>
   <221> MOD_RES
   <222> (14)..(14)
   <223> C' amidated
<400> 50
   Arg Arg Xaa Cys Tyr Xaa Arg Xaa Pro Tyr Arg Xaa Cys Arg
   1               5                   10
<210> 51
   <211> 14
   <212> PRT
   <213> Artificial
<220>
   <223> SYNTHETIC PEPTIDE
<220>
   <221> MOD_RES
   <222> (1)..(1)
   <223> N' ACETYLATED
<220>
   <221> MOD_RES
   <222> (3)..(3)
   <223> 3-((2-naphthyl) alanine
<220>
   <221> MOD_RES
   <222> (6)..(6)
   <223> citrulline
<220>
   <221> MOD_RES
   <222> (8)..(8)
   <223> D-lysine
<220>
   <221> MOD_RES
   <222> (12)..(12)
   <223> citrulline
<220>
   <221> MOD_RES
   <222> (14)..(14)
   <223> C' AMIDATED
<400> 51
   Arg Arg Xaa Cys Tyr Xaa Lys Xaa Pro Tyr Arg Xaa Cys Arg
   1               5                   10
<210> 52
   <211> 14
   <212> PRT
   <213> Artificial
<220>
   <223> synthetic peptide
<220>
   <221> MOD_RES
   <222> (1)..(1)
   <223> N' ACETYLATED
<220>
   <221> MOD_RES
   <222> (3)..(3)
   <223> 3-((2-naphthyl) alanine
<220>
   <221> MOD_RES
   <222> (8)..(8)
   <223> D-citrulline
<220>
   <221> MOD_RES
   <222> (12)..(12)
   <223> citrulline
<220>
   <221> MOD_RES
   <222> (14)..(14)
   <223> C' AMIDATED
<400> 52
   Arg Arg Xaa Cys Tyr Arg Lys Xaa Pro Tyr Arg Xaa Cys Arg
   1 5 10
<210> 53
   <211> 14
   <212> PRT
   <213> Artificial
<220>
   <223> synthetic peptide
<220>
   <221> MOD_RES
   <222> (1)..(1)
   <223> 4-fluorobenzoyl-arginine
<220>
   <221> MOD_RES
   <222> (3)..(3)
   <223> 3-((2-naphthyl) alanine
<220>
   <221> MOD_RES
   <222> (6)..(6)
   <223> citrulline
<220>
   <221> MOD_RES
   <222> (8)..(8)
   <223> D-glutamic acid
<220>
   <221> MOD_RES
   <222> (12)..(12)
   <223> citrulline
<220>
   <221> MISC_FEATURE
   <222> (14)..(14)
   <223> derivatization by a NH-methyl group
<400> 53
   Xaa Arg Xaa Cys Tyr Xaa Lys Xaa Pro Tyr Arg Xaa Cys Arg
   1 5 10
<210> 54
   <211> 14
   <212> PRT
   <213> Artificial
<220>
   <223> synthetic peptide
<220>
   <221> MOD_RES
   <222> (1)..(1)
   <223> 4-fluorobenzoyl-arginine
<220>
   <221> MOD_RES
   <222> (3)..(3)
   <223> 3-((2-naphthyl) alanine
<220>
   <221> MOD_RES
   <222> (6)..(6)
   <223> citrulline
<220>
   <221> MOD_RES
   <222> (8)..(8)
   <223> D-glutamic acid
<220>
   <221> MOD_RES
   <222> (12)..(12)
   <223> citrulline
<220>
   <221> MISC_FEATURE
   <222> (14)..(14)
   <223> derivatization by a NH-ethyl group
<400> 54
   Xaa Arg Xaa Cys Tyr Xaa Lys Xaa Pro Tyr Arg Xaa Cys Arg
   1               5                   10
<210> 55
   <211> 14
   <212> PRT
   <213> Artificial
<220>
   <223> synthetic peptide
<220>
   <221> MOD_RES
   <222> (1)..(1)
   <223> 4-fluorobenzoyl-arginine
<220>
   <221> MOD_RES
   <222> (3)..(3)
   <223> 3-((2-naphthyl) alanine
<220>
   <221> MOD_RES
   <222> (6)..(6)
   <223> citrulline
<220>
   <221> MOD_RES
   <222> (8)..(8)
   <223> D-glutamic acid
<220>
   <221> MOD_RES
   <222> (12)..(12)
   <223> citrulline
<220>
   <221> MISC_FEATURE
   <222> (14)..(14)
   <223> derivatization by NH-isopropyl
<400> 55
   Xaa Arg Xaa Cys Tyr Xaa Lys Xaa Pro Tyr Arg Xaa Cys Arg
   1               5                   10
<210> 56
   <211> 14
   <212> PRT
   <213> Artificial
<220>
   <223> synthetic peptide
<220>
   <221> MOD_RES
   <222> (1)..(1)
   <223> 4-fluorobenzoyl-arginine
<220>
   <221> MOD_RES
   <222> (3)..(3)
   <223> 3-((2-naphthyl) alanine
<220>
   <221> MOD_RES
   <222> (6)..(6)
   <223> citrulline
<220>
   <221> MOD_RES
   <222> (8)..(8)
   <223> D-glutamic acid
<220>
   <221> MOD_RES
   <222> (12)..(12)
   <223> citrulline
<220>
   <221> MISC_FEATURE
   <222> (14)..(14)
   <223> derivatization with a tyramine residue
<400> 56
   Xaa Arg Xaa Cys Tyr Xaa Lys Xaa Pro Tyr Arg Xaa Cys Arg
   1               5                   10
<210> 57
   <211> 14
   <212> PRT
   <213> Artificial
<220>
   <223> synthetic peptide
<220>
   <221> MOD_RES
   <222> (3)..(3)
   <223> 3-((2-naphthyl) alanine
<220>
   <221> MOD_RES
   <222> (8)..(8)
   <223> D-lysine
<220>
   <221> MOD_RES
   <222> (12)..(12)
   <223> citrulline
<400> 57
   Ala Arg Xaa Cys Tyr Arg Lys Xaa Pro Tyr Arg Xaa Cys Arg
   1               5                   10
<210> 58
   <211> 14
   <212> PRT
   <213> Artificial
<220>
   <223> synthetic peptide
<220>
   <221> MOD_RES
   <222> (3)..(3)
   <223> 3-((2-naphthyl) alanine
<220>
   <221> MOD_RES
   <222> (8)..(8)
   <223> D-lysine
<220>
   <221> MOD_RES
   <222> (12)..(12)
   <223> citrulline
<400> 58
   Arg Arg Xaa Cys Tyr Ala Lys Xaa Pro Tyr Arg Xaa Cys Arg
   1               5                   10
<210> 59
   <211> 14
   <212> PRT
   <213> Artificial
<220>
   <223> synthetic peptide
<220>
   <221> MOD_RES
   <222> (3)..(3)
   <223> 3-((2-naphthyl) alanine
<220>
   <221> MOD_RES
   <222> (8)..(8)
   <223> D-lysine
<220>
   <221> MOD_RES
   <222> (12)..(12)
   <223> citrulline
<400> 59
   Arg Arg Xaa Cys Tyr Arg Ala Xaa Pro Tyr Arg Xaa Cys Arg
   1               5                   10
<210> 60
   <211> 14
   <212> PRT
   <213> Artificial
<220>
   <223> synthetic peptide
<220>
   <221> MOD_RES
   <222> (3)..(3)
   <223> 3-((2-naphthyl) alanine
<220>
   <221> MOD_RES
   <222> (8)..(8)
   <223> D-alanine
<220>
   <221> MOD_RES
   <222> (12)..(12)
   <223> citrulline
<400> 60
   Arg Arg Xaa Cys Tyr Arg Lys Xaa Pro Tyr Arg Xaa Cys Arg
   1               5                   10
<210> 61
   <211> 14
   <212> PRT
   <213> Artificial
<220>
   <223> synthetic peptide
<220>
   <221> MOD_RES
   <222> (3)..(3)
   <223> 3-((2-naphthyl) alanine
<220>
   <221> MOD_RES
   <222> (8)..(8)
   <223> D-lysine
<220>
   <221> MOD_RES
   <222> (12)..(12)
   <223> citrulline
<400> 61
   Arg Arg Xaa Cys Tyr Arg Lys Xaa Ala Tyr Arg Xaa Cys Arg
   1               5                   10
<210> 62
   <211> 14
   <212> PRT
   <213> Artificial
<220>
   <223> synthetic peptide
<220>
   <221> MOD_RES
   <222> (3)..(3)
   <223> 3-((2-naphthyl) alanine
<220>
   <221> MOD_RES
   <222> (8)..(8)
   <223> D-lysine
<220>
   <221> MOD_RES
   <222> (12)..(12)
   <223> citrulline
<400> 62
   Arg Arg Xaa Cys Tyr Arg Lys Xaa Pro Ala Arg Xaa Cys Arg
   1               5                   10
<210> 63
   <211> 14
   <212> PRT
   <213> Artificial
<220>
   <223> synthetic peptide
<220>
   <221> MOD_RES
   <222> (3)..(3)
   <223> 3-((2-naphthyl) alanine
<220>
   <221> MOD_RES
   <222> (8)..(8)
   <223> D-lysine
<220>
   <221> MOD_RES
   <222> (12)..(12)
   <223> citrulline
<400> 63
   Arg Arg Xaa Cys Tyr Arg Lys Xaa Pro Tyr Ala Xaa Cys Arg
   1               5                   10
<210> 64
   <211> 14
   <212> PRT
   <213> Artificial
<220>
   <223> synthetic peptide
<220>
   <221> MOD_RES
   <222> (1)..(1)
   <223> citrulline
<220>
   <221> MOD_RES
   <222> (3)..(3)
   <223> 3-((2-naphthyl) alanine
<220>
   <221> MOD_RES
   <222> (8)..(8)
   <223> D-lysine
<220>
   <221> MOD_RES
   <222> (12)..(12)
   <223> citrulline
<400> 64
   Xaa Arg Xaa Cys Tyr Arg Lys Xaa Pro Tyr Arg Xaa Cys Arg
   1               5                   10
<210> 65
   <211> 14
   <212> PRT
   <213> Artificial
<220>
   <223> synthetic peptide
<220>
   <221> MOD_RES
   <222> (3)..(3)
   <223> 3-((2-naphthyl) alanine
<220>
   <221> MOD_RES
   <222> (6)..(6)
   <223> citrulline
<220>
   <221> MOD_RES
   <222> (8)..(8)
   <223> D-lysine
<220>
   <221> MOD_RES
   <222> (12)..(12)
   <223> citrulline
<400> 65
   Arg Arg Xaa Cys Tyr Xaa Lys Xaa Pro Tyr Arg Xaa Cys Arg
   1               5                   10
<210> 66
   <211> 14
   <212> PRT
   <213> Artificial
<220>
   <223> synthetic peptide
<220>
   <221> MOD_RES
   <222> (3).. (3)
   <223> 3-((2-naphthyl) alanine
<220>
   <221> MOD_RES
   <222> (6)..(6)
   <223> citrulline
<220>
   <221> MOD_RES
   <222> (8)..(8)
   <223> D-lysine
<220>
   <221> MOD_RES
   <222> (12)..(12)
   <223> citrulline
<220>
   <221> MOD_RES
   <222> (14)..(14)
   <223> C' AMIDATED
<400> 66
   Arg Arg Xaa Cys Tyr Xaa Lys Xaa Pro Tyr Arg Xaa Cys Arg
   1               5                   10
<210> 67
   <211> 14
   <212> PRT
   <213> Artificial
<220>
   <223> synthetic peptide
<220>
   <221> MOD_RES
   <222> (3)..(3)
   <223> 3-((2-naphthyl) alanine
<220>
   <221> MOD_RES
   <222> (7).. (7)
   <223> citrulline
<220>
   <221> MOD_RES
   <222> (8)..(8)
   <223> D-lysine
<220>
   <221> MOD_RES
   <222> (12)..(12)
   <223> citrulline
<400> 67
   Arg Arg Xaa Cys Tyr Arg Xaa Xaa Pro Tyr Arg Xaa Cys Arg
   1               5                   10
<210> 68
   <211> 14
   <212> PRT
   <213> Artificial
<220>
   <223> synthetic peptide
<220>
   <221> MOD_RES
   <222> (3)..(3)
   <223> 3-((2-naphthyl) alanine
<220>
   <221> MOD_RES
   <222> (6)..(6)
   <223> citrulline
<220>
   <221> MOD_RES
   <222> (8)..(8)
   <223> D-citrulline
<220>
   <221> MOD_RES
   <222> (12)..(12)
   <223> citrulline
<220>
   <221> MOD_RES
   <222> (14)..(14)
   <223> C' AMIDATED
<400> 68
   Arg Arg Xaa Cys Tyr Xaa Lys Xaa Pro Tyr Arg Xaa Cys Arg
   1               5                   10
<210> 69
   <211> 14
   <212> PRT
   <213> Artificial
<220>
   <223> synthetic peptide
<220>
   <221> MOD_RES
   <222> (3)..(3)
   <223> 3-((2-naphthyl) alanine
<220>
   <221> MOD_RES
   <222> (8)..(8)
   <223> D-lysine
<220>
   <221> MOD_RES
   <222> (12)..(12)
   <223> citrulline
<400> 69
   Arg Arg Xaa Cys Tyr Arg Lys Xaa Pro Tyr Arg Xaa Cys Arg
   1               5                   10
<210> 70
   <211> 14
   <212> PRT
   <213> Artificial
<220>
   <223> synthetic peptide
<220>
   <221> MOD_RES
   <222> (3)..(3)
   <223> 3-((2-naphthyl) alanine
<220>
   <221> MOD_RES
   <222> (6).. (6)
   <223> citrulline
<220>
   <221> MOD_RES
   <222> (8)..(8)
   <223> D-citrulline
<220>
   <221> MOD_RES
   <222> (12)..(12)
   <223> citrulline
<400> 70
   Arg Arg Xaa Cys Tyr Xaa Lys Xaa Pro Tyr Arg Xaa Cys Arg
   1               5                   10
<210> 71
   <211> 14
   <212> PRT
   <213> Artificial
<220>
   <223> synthetic peptide
<220>
   <221> MOD_RES
   <222> (3)..(3)
   <223> 3-((2-naphthyl) alanine
<220>
   <221> MOD_RES
   <222> (8)..(8)
   <223> D-citrulline
<220>
   <221> MOD_RES
   <222> (12)..(12)
   <223> citrulline
<400> 71
   Arg Arg Xaa Cys Tyr Arg Lys Xaa Pro Tyr Arg Xaa Cys Arg
   1               5                   10
<210> 72
   <211> 14
   <212> PRT
   <213> Artificial
<220>
   <223> synthetic peptide
<220>
   <221> MOD_RES
   <222> (1)..(1)
   <223> citrulline
<220>
   <221> MOD_RES
   <222> (3)..(3)
   <223> 3-((2-naphthyl) alanine
<220>
   <221> MOD_RES
   <222> (8)..(8)
   <223> D-citrulline
<220>
   <221> MOD_RES
   <222> (12)..(12)
   <223> citrulline
<220>
   <221> MOD_RES
   <222> (14)..(14)
   <223> C' Amidated
<400> 72
   Xaa Arg Xaa Cys Tyr Arg Lys Xaa Pro Tyr Arg Xaa Cys Arg
   1               5                   10
<210> 73
   <211> 2982
   <212> DNA
   <213> Homo sapiens
<400> 73
   atgccggcgt tggcgcgcga cggcggccag ctgccgctgc tcgttgtttt ttctgcaatg      60
   atatttggga ctattacaaa tcaagatctg cctgtgatca agtgtgtttt aatcaatcat     120
   aagaacaatg attcatcagt ggggaagtca tcatcatatc ccatggtatc agaatccccg     180
   gaagacctcg ggtgtgcgtt gagaccccag agctcaggga cagtgtacga agctgccgct     240
   gtggaagtgg atgtatctgc ttccatcaca ctgcaagtgc tggtcgacgc cccagggaac     300
   atttcctgtc tctgggtctt taagcacagc tccctgaatt gccagccaca ttttgattta     360
   caaaacagag gagttgtttc catggtcatt ttgaaaatga cagaaaccca agctggagaa     420
   tacctacttt ttattcagag tgaagctacc aattacacaa tattgtttac agtgagtata     480
   agaaataccc tgctttacac attaagaaga ccttacttta gaaaaatgga aaaccaggac     540
   gccctggtct gcatatctga gagcgttcca gagccgatcg tggaatgggt gctttgcgat     600
   tcacaggggg aaagctgtaa agaagaaagt ccagctgttg ttaaaaagga ggaaaaagtg     660
   cttcatgaat tatttgggac ggacataagg tgctgtgcca gaaatgaact gggcagggaa     720
   tgcaccaggc tgttcacaat agatctaaat caaactcctc agaccacatt gccacaatta     780
   tttcttaaag taggggaacc cttatggata aggtgcaaag ctgttcatgt gaaccatgga     840
   ttcgggctca cctgggaatt agaaaacaaa gcactcgagg agggcaacta ctttgagatg     900
   agtacctatt caacaaacag aactatgata cggattctgt ttgcttttgt atcatcagtg     960
   gcaagaaacg acaccggata ctacacttgt tcctcttcaa agcatcccag tcaatcagct    1020
   ttggttacca tcgtagaaaa gggatttata aatgctacca attcaagtga agattatgaa    1080
   attgaccaat atgaagagtt ttgtttttct gtcaggttta aagcctaccc acaaatcaga     1140
   tgtacgtgga ccttctctcg aaaatcattt ccttgtgagc aaaagggtct tgataacgga     1200
   tacagcatat ccaagttttg caatcataag caccagccag gagaatatat attccatgca     1260
   gaaaatgatg atgcccaatt taccaaaatg ttcacgctga atataagaag gaaacctcaa     1320
   gtgctcgcag aagcatcggc aagtcaggcg tcctgtttct cggatggata cccattacca     1380
   tcttggacct ggaagaagtg ttcagacaag tctcccaact gcacagaaga gatcacagaa     1440
   ggagtctgga atagaaaggc taacagaaaa gtgtttggac agtgggtgtc gagcagtact     1500
   ctaaacatga gtgaagccat aaaagggttc ctggtcaagt gctgtgcata caattccctt     1560
   ggcacatctt gtgagacgat ccttttaaac tctccaggcc ccttcccttt catccaagac     1620
   aacatctcat tctatgcaac aattggtgtt tgtctcctct tcattgtcgt tttaaccctg     1680
   ctaatttgtc acaagtacaa aaagcaattt aggtatgaaa gccagctaca gatggtacag     1740
   gtgaccggct cctcagataa tgagtacttc tacgttgatt tcagagaata tgaatatgat     1800
   ctcaaatggg agtttccaag agaaaattta gagtttggga aggtactagg atcaggtgct     1860
   tttggaaaag tgatgaacgc aacagcttat ggaattagca aaacaggagt ctcaatccag     1920
   gttgccgtca aaatgctgaa agaaaaagca gacagctctg aaagagaggc actcatgtca     1980
   gaactcaaga tgatgaccca gctgggaagc cacgagaata ttgtgaacct gctgggggcg     2040
   tgcacactgt caggaccaat ttacttgatt tttgaatact gttgctatgg tgatcttctc     2100
   aactatctaa gaagtaaaag agaaaaattt cacaggactt ggacagagat tttcaaggaa     2160
   cacaatttca gtttttaccc cactttccaa tcacatccaa attccagcat gcctggttca     2220
   agagaagttc agatacaccc ggactcggat caaatctcag ggcttcatgg gaattcattt     2280
   cactctgaag atgaaattga atatgaaaac caaaaaaggc tggaagaaga ggaggacttg     2340
   aatgtgctta catttgaaga tcttctttgc tttgcatatc aagttgccaa aggaatggaa     2400
   tttctggaat ttaagtcgtg tgttcacaga gacctggccg ccaggaacgt gcttgtcacc     2460
   cacgggaaag tggtgaagat atgtgacttt ggattggctc gagatatcat gagtgattcc     2520
   aactatgttg tcaggggcaa tgcccgtctg cctgtaaaat ggatggcccc cgaaagcctg     2580
   tttgaaggca tctacaccat taagagtgat gtctggtcat atggaatatt actgtgggaa     2640
   atcttctcac ttggtgtgaa tccttaccct ggcattccgg ttgatgctaa cttctacaaa     2700
   ctgattcaaa atggatttaa aatggatcag ccattttatg ctacagaaga aatatacatt     2760
   ataatgcaat cctgctgggc ttttgactca aggaaacggc catccttccc taatttgact     2820
   tcgtttttag gatgtcagct ggcagatgca gaagaagcga tgtatcagaa tgtggatggc     2880
   cgtgtttcgg aatgtcctca cacctaccaa aacaggcgac ctttcagcag agagatggat     2940
   ttggggctac tctctccgca ggctcaggtc gaagattcgt ag                        2982
<210> 74
   <211> 993
   <212> PRT
   <213> Homo sapiens
<400> 74
   Met Pro Ala Leu Ala Arg Asp Gly Gly Gln Leu Pro Leu Leu Val Val
   1               5                   10                  15
   Phe Ser Ala Met Ile Phe Gly Thr Ile Thr Asn Gln Asp Leu Pro Val
               20                  25                  30
   Ile Lys Cys Val Leu Ile Asn His Lys Asn Asn Asp Ser Ser Val Gly
           35                  40                  45
   Lys Ser Ser Ser Tyr Pro Met Val Ser Glu Ser Pro Glu Asp Leu Gly
       50                  55                  60
   Cys Ala Leu Arg Pro Gln Ser Ser Gly Thr Val Tyr Glu Ala Ala Ala
   65                  70                  75                  80
   Val Glu Val Asp Val Ser Ala Ser Ile Thr Leu Gln Val Leu Val Asp
                   85                  90                  95
   Ala Pro Gly Asn Ile Ser Cys Leu Trp Val Phe Lys His Ser Ser Leu
               100                 105                 110
   Asn Cys Gln Pro His Phe Asp Leu Gln Asn Arg Gly Val Val Ser Met
           115                 120                 125
   Val Ile Leu Lys Met Thr Glu Thr Gln Ala Gly Glu Tyr Leu Leu Phe
       130                 135                 140
   Ile Gln Ser Glu Ala Thr Asn Tyr Thr Ile Leu Phe Thr Val Ser Ile
   145                 150                 155                 160
   Arg Asn Thr Leu Leu Tyr Thr Leu Arg Arg Pro Tyr Phe Arg Lys Met
                   165                 170                 175
   Glu Asn Gln Asp Ala Leu Val Cys Ile Ser Glu Ser Val Pro Glu Pro
               180                 185                 190
   Ile Val Glu Trp Val Leu Cys Asp Ser Gln Gly Glu Ser Cys Lys Glu
           195                 200                 205
   Glu Ser Pro Ala Val Val Lys Lys Glu Glu Lys Val Leu His Glu Leu
       210                 215                 220
   Phe Gly Thr Asp Ile Arg Cys Cys Ala Arg Asn Glu Leu Gly Arg Glu
   225                 230                 235                 240
   Cys Thr Arg Leu Phe Thr Ile Asp Leu Asn Gln Thr Pro Gln Thr Thr
                   245                 250                 255
   Leu Pro Gln Leu Phe Leu Lys Val Gly Glu Pro Leu Trp Ile Arg Cys
               260                 265                 270
   Lys Ala Val His Val Asn His Gly Phe Gly Leu Thr Trp Glu Leu Glu
           275                 280                 285
   Asn Lys Ala Leu Glu Glu Gly Asn Tyr Phe Glu Met Ser Thr Tyr Ser
       290                 295                 300
   Thr Asn Arg Thr Met Ile Arg Ile Leu Phe Ala Phe Val Ser Ser Val
   305                 310                 315                 320
   Ala Arg Asn Asp Thr Gly Tyr Tyr Thr Cys Ser Ser Ser Lys His Pro
                   325                 330                 335
   Ser Gln Ser Ala Leu Val Thr Ile Val Glu Lys Gly Phe Ile Asn Ala
               340                 345                 350
   Thr Asn Ser Ser Glu Asp Tyr Glu Ile Asp Gln Tyr Glu Glu Phe Cys
           355                 360                 365
   Phe Ser Val Arg Phe Lys Ala Tyr Pro Gln Ile Arg Cys Thr Trp Thr
       370                 375                 380
   Phe Ser Arg Lys Ser Phe Pro Cys Glu Gln Lys Gly Leu Asp Asn Gly
   385                 390                 395                 400
   Tyr Ser Ile Ser Lys Phe Cys Asn His Lys His Gln Pro Gly Glu Tyr
                   405                 410                 415
   Ile Phe His Ala Glu Asn Asp Asp Ala Gln Phe Thr Lys Met Phe Thr
               420                 425                 430
   Leu Asn Ile Arg Arg Lys Pro Gln Val Leu Ala Glu Ala Ser Ala Ser
           435                 440                 445
   Gln Ala Ser Cys Phe Ser Asp Gly Tyr Pro Leu Pro Ser Trp Thr Trp
       450                 455                 460
   Lys Lys Cys Ser Asp Lys Ser Pro Asn Cys Thr Glu Glu Ile Thr Glu
   465                 470                 475                 480
   Gly Val Trp Asn Arg Lys Ala Asn Arg Lys Val Phe Gly Gln Trp Val
                   485                 490                 495
   Ser Ser Ser Thr Leu Asn Met Ser Glu Ala Ile Lys Gly Phe Leu Val
               500                 505                 510
   Lys Cys Cys Ala Tyr Asn Ser Leu Gly Thr Ser Cys Glu Thr Ile Leu
           515                 520                 525
   Leu Asn Ser Pro Gly Pro Phe Pro Phe Ile Gln Asp Asn Ile Ser Phe
       530                 535                 540
   Tyr Ala Thr Ile Gly Val Cys Leu Leu Phe Ile Val Val Leu Thr Leu
   545                 550                 555                 560
   Leu Ile Cys His Lys Tyr Lys Lys Gln Phe Arg Tyr Glu Ser Gln Leu
                   565                 570                 575
   Gln Met Val Gln Val Thr Gly Ser Ser Asp Asn Glu Tyr Phe Tyr Val
               580                 585                 590
   Asp Phe Arg Glu Tyr Glu Tyr Asp Leu Lys Trp Glu Phe Pro Arg Glu
           595                 600                 605
   Asn Leu Glu Phe Gly Lys Val Leu Gly Ser Gly Ala Phe Gly Lys Val
       610                 615                 620
   Met Asn Ala Thr Ala Tyr Gly Ile Ser Lys Thr Gly Val Ser Ile Gln
   625                 630                 635                 640
   Val Ala Val Lys Met Leu Lys Glu Lys Ala Asp Ser Ser Glu Arg Glu
                   645                 650                 655
   Ala Leu Met Ser Glu Leu Lys Met Met Thr Gln Leu Gly Ser His Glu
               660                 665                 670
   Asn Ile Val Asn Leu Leu Gly Ala Cys Thr Leu Ser Gly Pro Ile Tyr
           675                 680                 685
   Leu Ile Phe Glu Tyr Cys Cys Tyr Gly Asp Leu Leu Asn Tyr Leu Arg
       690                 695                 700
   Ser Lys Arg Glu Lys Phe His Arg Thr Trp Thr Glu Ile Phe Lys Glu
   705                 710                 715                 720
   His Asn Phe Ser Phe Tyr Pro Thr Phe Gln Ser His Pro Asn Ser Ser
                   725                 730                 735
   Met Pro Gly Ser Arg Glu Val Gln Ile His Pro Asp Ser Asp Gln Ile
               740                 745                 750
   Ser Gly Leu His Gly Asn Ser Phe His Ser Glu Asp Glu Ile Glu Tyr
           755                 760                 765
   Glu Asn Gln Lys Arg Leu Glu Glu Glu Glu Asp Leu Asn Val Leu Thr
       770                 775                 780
   Phe Glu Asp Leu Leu Cys Phe Ala Tyr Gln Val Ala Lys Gly Met Glu
   785                 790                 795                 800
   Phe Leu Glu Phe Lys Ser Cys Val His Arg Asp Leu Ala Ala Arg Asn
                   805                 810                 815
   Val Leu Val Thr His Gly Lys Val Val Lys Ile Cys Asp Phe Gly Leu
               820                 825                 830
   Ala Arg Asp Ile Met Ser Asp Ser Asn Tyr Val Val Arg Gly Asn Ala
           835                 840                 845
   Arg Leu Pro Val Lys Trp Met Ala Pro Glu Ser Leu Phe Glu Gly Ile
       850                 855                 860
   Tyr Thr Ile Lys Ser Asp Val Trp Ser Tyr Gly Ile Leu Leu Trp Glu
   865                 870                 875                 880
   Ile Phe Ser Leu Gly Val Asn Pro Tyr Pro Gly Ile Pro Val Asp Ala
                   885                 890                 895
   Asn Phe Tyr Lys Leu Ile Gln Asn Gly Phe Lys Met Asp Gln Pro Phe
               900                 905                 910
   Tyr Ala Thr Glu Glu Ile Tyr Ile Ile Met Gln Ser Cys Trp Ala Phe
           915                 920                 925
   Asp Ser Arg Lys Arg Pro Ser Phe Pro Asn Leu Thr Ser Phe Leu Gly
       930                 935                 940
   Cys Gln Leu Ala Asp Ala Glu Glu Ala Met Tyr Gln Asn Val Asp Gly
   945                 950                 955                 960
   Arg Val Ser Glu Cys Pro His Thr Tyr Gln Asn Arg Arg Pro Phe Ser
                   965                 970                 975
   Arg Glu Met Asp Leu Gly Leu Leu Ser Pro Gln Ala Gln Val Glu Asp
               980                 985                 990
   Ser

## Description

### FIELD AND BACKGROUND OF THE INVENTION

The present disclosure relates to methods of treating acute myeloid leukemia (AML) and, more particularly, to the use of a CXCR4-antagonistic peptide in the treatment of AML with a FLT3 mutation.

AML is a heterogeneous group of diseases characterized by the uncontrolled proliferation of hematopoietic stem cells and progenitors (blasts) with a reduced capacity to differentiate into mature cells (Estey et al., Lancet 368:1894-1907, 2006). While many patients with AML are able to achieve a complete remission (CR) with traditional chemotherapy, the majority of AML patients eventually relapse. Rates of relapse are particularly high for patients with a FLT3 (FMS-like tyrosine kinase 3) internal tandem duplication (ITD) mutation. FLT3-ITD mutations are found in about a quarter of the AML patients (Levis and Small, Leukemia 17: 1738-1752, 2003). Several FLT3 inhibitors are currently under clinical investigations but none has yet been approved for the treatment of AML with a FLT3 mutation (Fathi and Chen, Am. J. Blood Res. 1:175-189,2011).

The bicyclam drug termed AMD3100, originally discovered as an anti-HIV compound, specifically interacts with CXCR4 in an antagonistic manner. Blocking CXCR4 receptor with AMD3100 results in the mobilization of hematopoietic progenitor cells. WO 2007/022523 discloses the use of CXCR4 agonists such as AMD3100 for enhancing the effectiveness of chemotherapeutic methods in subjects afflicted with myeloid or hematopoietic malignancies.

T-140 is a 14-residue synthetic peptide developed as a specific CXCR4 antagonist that suppress HIV-1 (X4-HIV-1) entry to T cells through specific binding to CXCR4 (Tamamura et al., Biochem. Biophys. Res. Commun. 253(3): 877-882, 1998). Subsequently, peptide analogs of T-140 were developed as specific CXCR4-antagonisic peptides with inhibitory activity at nanomolar levels [Tamamura et al. (Org. Biomol. Chem. 1: 3663-3669, 2003), WO 2002/020561, WO 2004/020462, WO 2004/087068, WO 00/09152, US 2002/0156034, and WO 2004/024178].

WO 2004/087068 discloses antagonists of chemokine receptors, particularly the CXCR4 receptor, and methods of their use, for example, in the treatment, prevention or diagnosis of cancer. The '068 publication discloses that exemplary CXCR4 peptide antagonists include T140 and derivatives of T140, and that the pathology includes cancer such as breast, brain, pancreatic, ovarian, prostate, kidney, and non-small lung cancer.

WO 00/09152 discloses a variety of therapeutic uses for CXCR4 antagonists such as in the treatment of cancer.

WO 2004/024178 discloses the use of a chemokine receptor antagonist as a ligand for the CXCR4 receptor for the apoptosis-inducing treatment and/or the prevention of the metastatic spread of cancer cells in a patient.

U.S. Publication No. 2002/0156034 discloses the use of CXCR4 antagonists for the treatment of hematopoietic cells such as in cancer.

WO 2002/020561 discloses peptide analogs and derivatives of T-140. The 561 publication demonstrates that the claimed peptides are potent CXCR4 inhibitors, manifesting high anti-HIV virus activity and low cytotoxicity.

Recently, a comparative study between the CXCR4 antagonists TN140 and AMD3100 suggested that TN140 is more effective than AMD3100 as a monotherapy in AML. TN140 and to a lesser extend AMD3100 induced regression of human CXCR4-expressing AML cells and targeted the NOD/Shi-scid/IL-2Rynull (NOG) leukemia-initiating cells (LICs) (Y. Zhang et al., Cell Death and Disease, 2012).

WO 2004/020462 discloses additional novel peptide analogs and derivatives of T-140, including 4F-benzoyl-TN14003. The '462 publication further discloses preventive and therapeutic compositions and methods of using same utilizing T-140 analogs for the treatment of cancer, such as T-Cell leukemia.

Beider et al. (Exp. Hematol. 39:282-92, 2011) reported that 4F-benzoyl-TN14003 exhibits a CXCR4-dependent preferential cytotoxicity toward malignant cells of hematopoietic origin including AML. In vivo, subcutaneous injections of 4F-benzoyl-TN14003 significantly reduced the growth of human AML xenografts.

### SUMMARY OF THE INVENTION

The present invention provides a novel safe and effective method for the treatment of acute myeloid leukemia with a FLT3 mutation.

According to an aspect there is provided a method of treating acute myeloid leukemia with a FLT3 mutation. The method includes the steps of (i) identifying a subject having AML with a FMS-like tyrosine kinase 3 (FLT3)-mutation and (ii) administrating to the subject a therapeutically effective amount of a CXCR4-antagonistic peptide.

According to an aspect there is provided a use of a CXCR4-antagonistic peptide in the manufacture of a medicament identified for the treatment of AML with a FMS-like tyrosine kinase 3 (FLT3) mutation in a subject in need thereof.

According to an aspect of the present invention there is provided a CXCR4-antagonistic peptide for the treatment of AML with a FMS-like tyrosine kinase 3 (FLT3) comprising a CXCR4-antagonistic peptide and a chemotherapeutic agent.

According to an aspect of the present invention there is provided an article of manufacture identified for the treatment of AML with a FMS-like tyrosine kinase 3 (FLT3) mutation comprising a CXCR4-antagonistic peptide and a chemotherapeutic agent.

According to further features in preferred embodiments of the invention described below, the CXCR4-antagonistic peptide and the chemotherapeutic agent are in separate containers.

According to further features in preferred embodiments of the invention described below, the FLT3 mutation is a FLT3 internal tandem duplication (ITD) mutation.

According to still further features in the described preferred embodiments the CXCR4-antagonistic peptide is as set forth in SEQ ID NO: 1.

According to still further features in the described preferred embodiments the CXCR4-antagonistic peptide is administered to the subject in a daily amount between 0.1 to 10 mg per kg of body weight.

According to still further features in the described preferred embodiments the CXCR4-antagonistic peptide is administered subcutaneously.

According to still further features in the described preferred embodiments the CXCR4-antagonistic peptide is administered intravenously.

According to still further features in the described preferred embodiments the method of treating acute myeloid leukemia further includes a step of administering to the subject a therapeutically effective amount of a chemotherapeutic agent.

According to still further features in the described preferred embodiments the chemotherapeutic agent is cytarabine (ARA-C).

According to still further features in the described preferred embodiments the chemotherapeutic agent is quizartinib (AC220).

According to still further features the chemotherapeutic agent synergizes with the CXCR4-antagonistic peptide in inducing apoptosis of AML cells.

The present invention successfully addresses the shortcomings of the presently known configurations by providing a novel method of treating acute myeloid leukemia that is safe and effective.

Unless otherwise defined, all technical and/or scientific terms used herein have the same meaning as commonly understood by one of ordinary skill in the art to which the invention pertains. Although methods and materials similar or equivalent to those described herein can be used in the practice or testing of embodiments of the invention, exemplary methods and/or materials are described below. In case of conflict, the patent specification, including definitions, will control. In addition, the materials, methods, and examples are illustrative only and are not intended to be necessarily limiting. The invention is defined in the claims.

### BRIEF DESCRIPTION OF THE DRAWINGS

Some embodiments of the invention are herein described, by way of example only, with reference to the accompanying drawings. With specific reference now to the drawings in detail, it is stressed that the particulars shown are by way of example and for purposes of illustrative discussion of embodiments of the invention. In this regard, the description taken with the drawings makes apparent to those skilled in the art how embodiments of the invention may be practiced.

In the drawings:

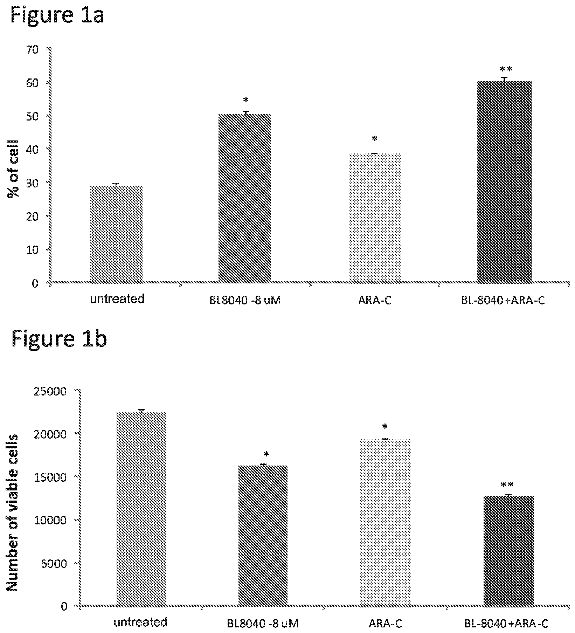

Figures 1a-b are bar graphs illustrating the effect of BL-8040 (8µM), ARA-C (50 ng/ml), or a combination thereof, on the survival of human primary AML cells with FLT3-ITD. FIG. 1A shows the incidence of dead cells. FIG. 1B shows the number of viable cells. A single asterisk (*) indicates a statistically significant difference (p < 0.05) vs. untreated control. A pair of asterisks (**) indicates a statistically significant difference (p < 0.01) vs. BL-8040 only.

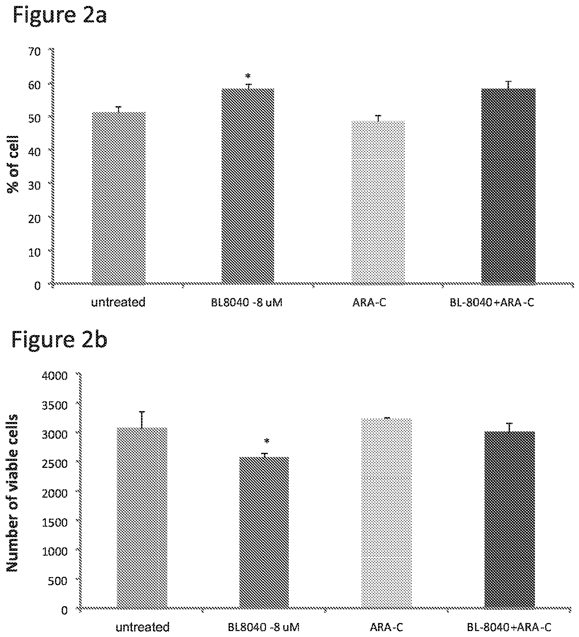

Figures 2a-b are bar graphs illustrating the effect of BL-8040 (8µM), ARA-C (50 ng/ml), or a combination thereof, on the survival of human primary AML cells with FLT3-WT. FIG. 2A shows the incidence of dead cells. FIG. 2B shows the number of viable cells. A single asterisk (*) indicates a statistically significant difference (p < 0.05) vs. untreated control.

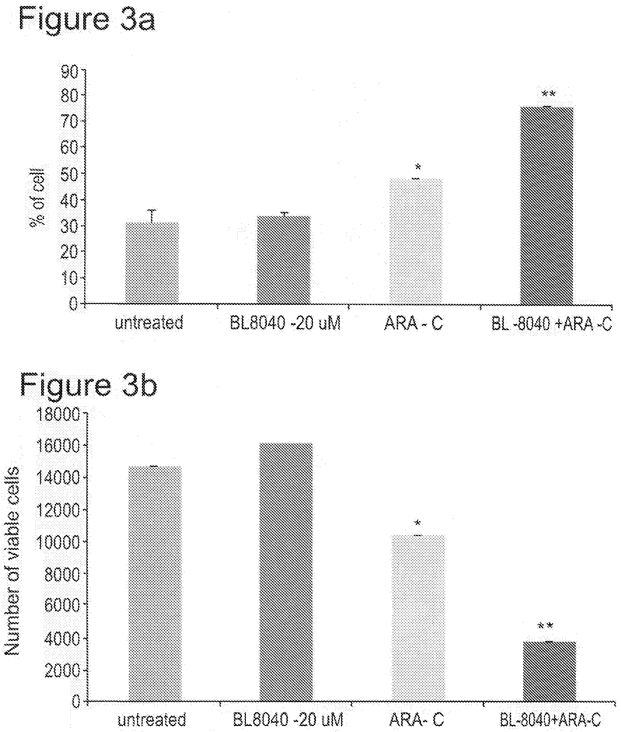

Figures 3a-b are bar graphs illustrating the effect of BL-8040 (20 µM), ARA-C (50 ng/ml), or a combination thereof, on the survival of MV4-11 human AML cells with FLT3-ITD. FIG. 3A shows the incidence of dead cells. FIG. 3B shows the number of viable cells. A single asterisk (*) indicates a statistically significant difference (p < 0.05) vs. untreated control. A pair of asterisks (**) indicates a statistically significant difference (p < 0.01) vs. ARA-C only.

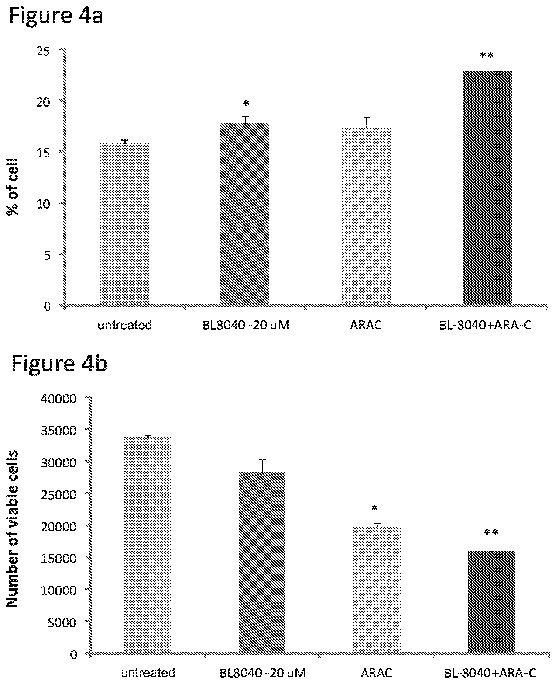

Figures 4a-b are bar graphs illustrating the effect of BL-8040 (20 µM), ARA-C (50 ng/ml), or a combination thereof, on the survival of HL60 human AML cells with FLT3-WT. FIG. 4A shows the incidence of dead cells. FIG. 4B shows the number of viable cells. A single asterisk (*) indicates a statistically significant difference (p < 0.05) vs. untreated control. A pair of asterisks (**) indicates a statistically significant difference (p < 0.01) vs. ARA-C only.

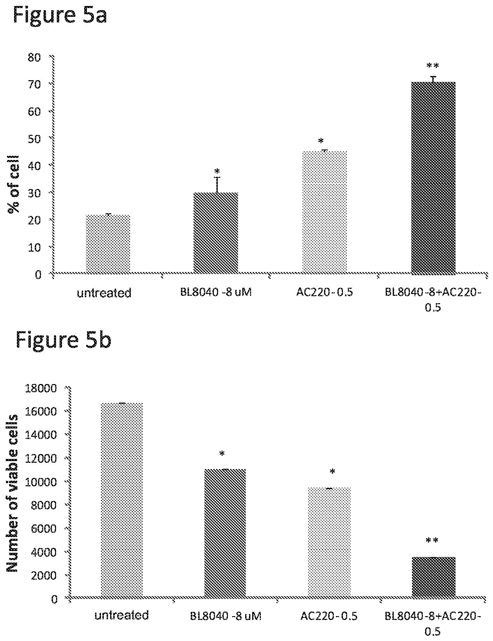

Figures 5a-b are bar graphs illustrating the effect of BL-8040 (8µM), AC220 (0.5 nM), or a combination thereof, on the survival of M4V-11human AML cells with FLT3-ITD. FIG. 5A shows the incidence of dead cells. FIG. 5B shows the number of viable cells. A single asterisk (*) indicates a statistically significant difference (p < 0.05) vs. untreated control. A pair of asterisks (**) indicates a statistically significant difference (p < 0.01) vs. AC220 only.

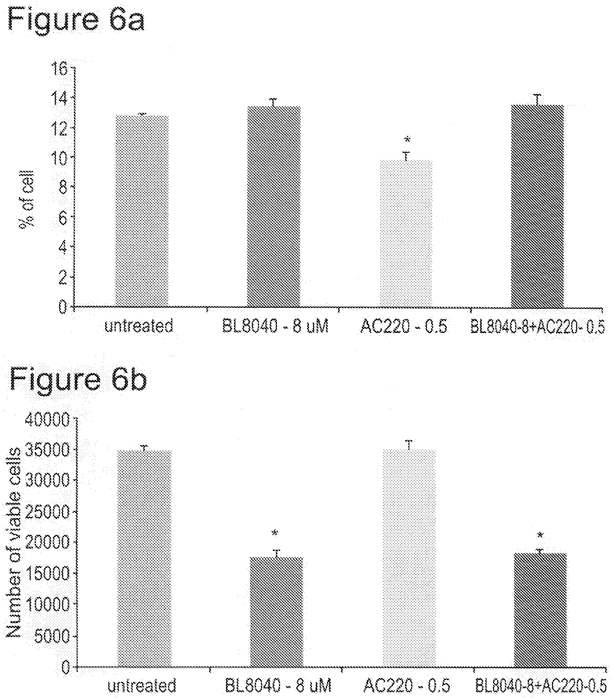

Figures 6a-b are bar graphs illustrating the effect of BL-8040 (8µM), AC220 (0.5 nM), or a combination thereof, on the survival of HL60 human AML cells with FLT3-WT. FIG. 6A shows the incidence of dead cells. FIG. 6B shows the number of viable cells. A single asterisk (*) indicates a statistically significant difference (p < 0.05) vs. untreated control.

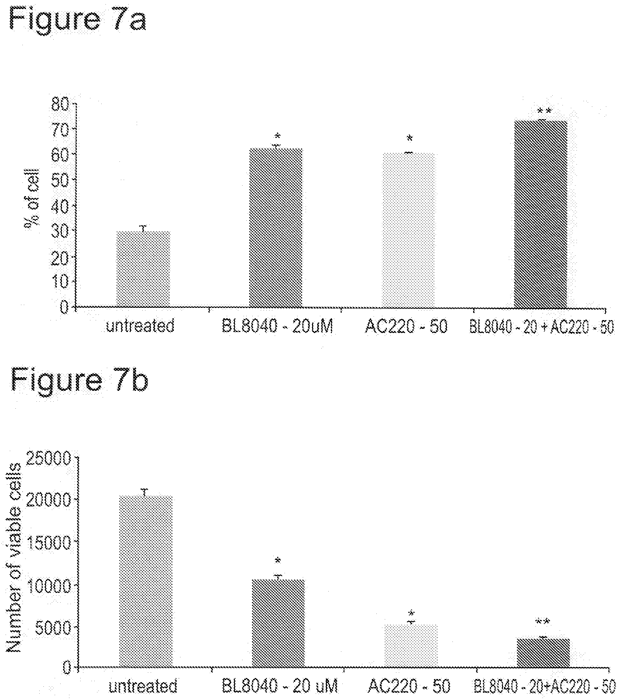

Figures 7a-b are bar graphs illustrating the effect of BL-8040 (20 µM), AC220 (50 nM), or a combination thereof, on the survival of human primary AML cells with FLT3-ITD. FIG. 7A shows the incidence of dead cells. FIG. 7B shows the number of viable cells. A single asterisk (*) indicates a statistically significant difference (p < 0.05) vs. untreated control. A pair of asterisks (**) indicates a statistically significant difference (p < 0.05) vs. AC220 only.

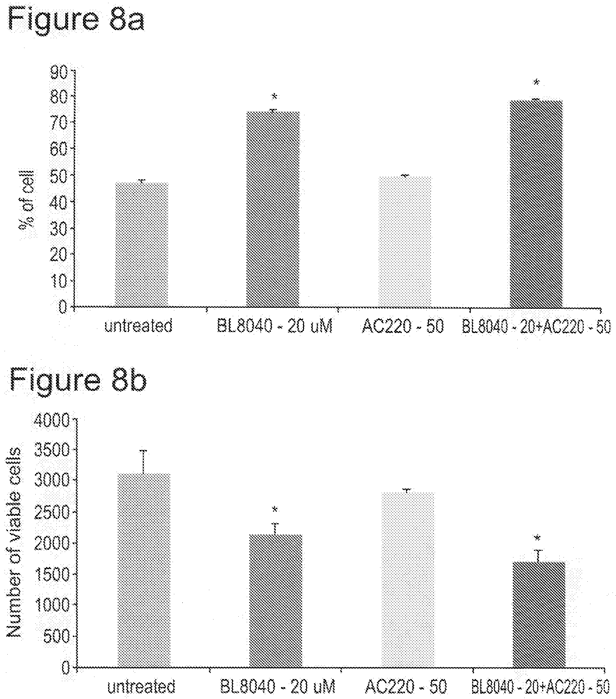

Figures 8a-b are bar graphs illustrating the effect of BL-8040 (20 µM), AC220 (50 nM), or a combination thereof, on the survival of human primary AML cells with FLT3-WT. FIG. 8A shows the incidence of dead cells. FIG. 8B shows the number of viable cells. A single asterisk (*) indicates a statistically significant difference (p < 0.05) vs. untreated control.

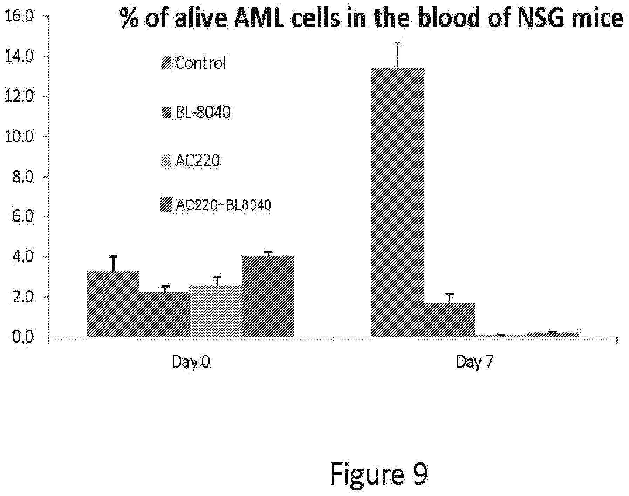

Figure 9 is a bar graph showing the percentage of live AML cells in the blood of NSG mice treated with BL-8040, AC220 or both.

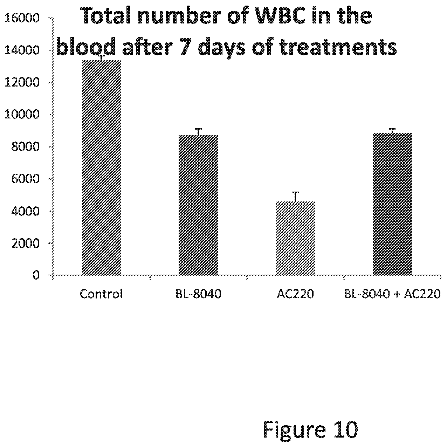

Figure 10 is a bar graph showing the total number of white blood cells in the blood of NSG mice treated with BL-8040, AC220 or both 7 days post treatment.

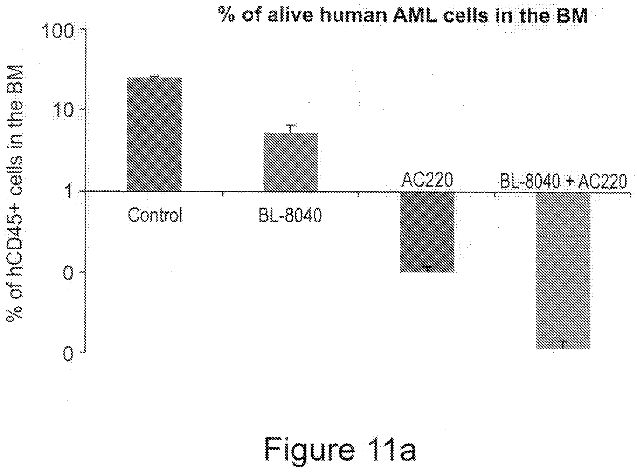

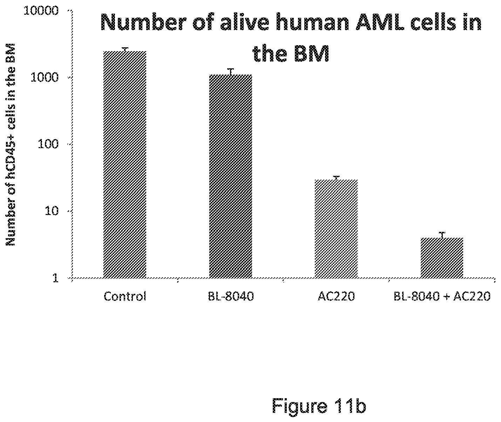

Figures 11a-b are bar graphs showing the percentage (Figure 11A) or number (Figure 11B) of live AML cells in the bone marrow (BM) of NSG mice treated with BL-8040, AC220 or both.

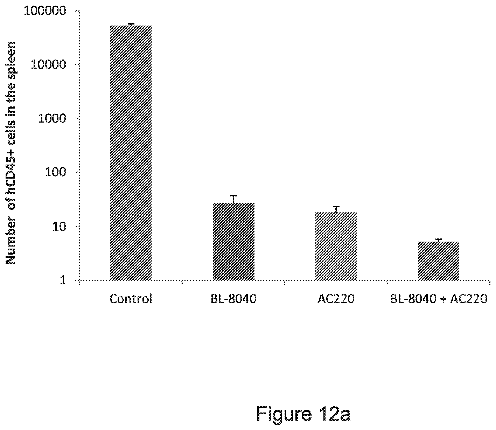

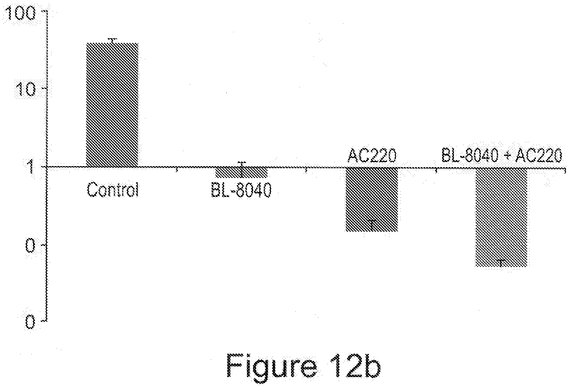

Figures 12a-b are bar graphs showing the number (Figure 12A) or percentage (Figure 12B) of live AML cells in the spleen of NSG mice treated with BL-8040, AC220 or both.

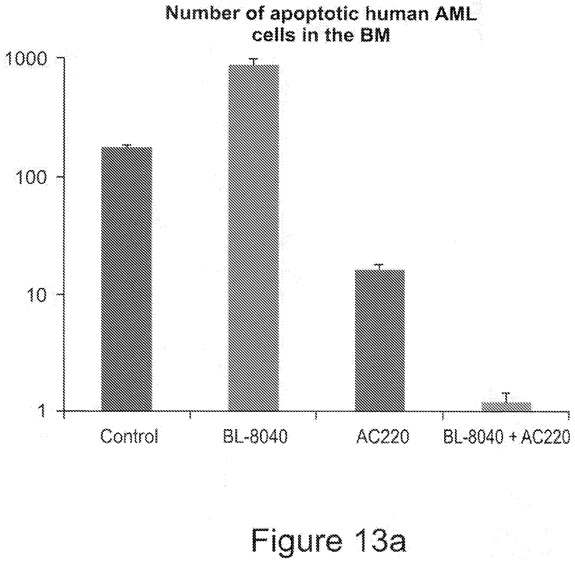

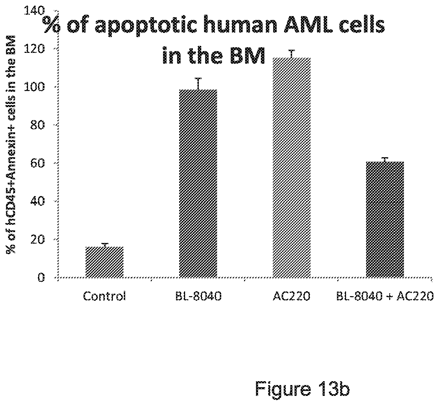

Figures 13a-b are bar graphs showing the number (Figure 13A) or percentage (Figure 13B) of apoptotic AML cells in the bone marrow (BM) of NSG mice treated with BL-8040, AC220 or both.

### DESCRIPTION OF SPECIFIC EMBODIMENTS OF THE INVENTION

The present disclosure in some embodiments thereof, relates to uses of CXCR4-antagonistic peptides in the treatment of acute myeloid leukemia with FLT3 mutations.

The principles and operation of the present invention may be better understood with reference to the drawings and accompanying descriptions.

Before explaining at least one embodiment of the invention in detail, it is to be understood that the invention is not necessarily limited in its application to the details set forth in the following description or exemplified by the Examples. The invention is capable of other embodiments or of being practiced or carried out in various ways. Also, it is to be understood that the phraseology and terminology employed herein is for the purpose of description and should not be regarded as limiting.

FLT3-ITD mutation is found in about a quarter of the patients having acute myeloid leukemia (AML) and it is considered to be a particularly poor prognosis (Levis and Small, Leukemia 17: 1738-1752, 2003).

While reducing the present invention to practice, the present inventors have surprisingly uncovered that a CXCR4-antagonistic peptide, BL-8040 (SEQ ID NO: 1), is substantially more toxic against AML cells with FLT3-ITD mutation, by comparison to wild-type AML cells (see in Example 1 hereinbelow).

Thus, according to an aspect of the present disclosure there is provided a method of treating acute myeloid leukemia (AML). The method includes the steps of (i) identifying a subject having AML with a FMS-like tyrosine kinase 3 (FLT3)-mutation and (ii) administrating to the subject a therapeutically effective amount of a CXCR4-antagonistic peptide.

Wild type FLT3 sequences are provided in SEQ ID NOs: 73 and 74.

Subjects having FLT3 mutations can be identified using methods known in the art such as described, for example, in Murphy et al., J Mol. Diagn. 5: 96-102, 2003. Mutations in FLT3 are also described in Markovic et al. J. Biochem. Cell Biol. 2005 37(6):1168-72; and Nakao et al. 1996 Leukemia 10(12):1911-8.

Internal tandem duplication in FLT3 gene is typically characterized by abbarent RNA transcripts which may stem from a simple internal duplication within exon 11; internal duplication (26 bp) with a 4-bp insertion; or a 136-bp sequence from the 3' part of exon 11 to intron 11 and the first 16-bp sequence of exon 12 are duplicated with 1-bp insertion (see Nakao, supra). Other abnormalities may also exist.

According to a specific embodiment, the FLT3 mutation results in activation of the protein.

In one embodiment the FLT3 mutation is a FLT3 internal-tandem duplication (ITD) mutation (Levis and Small, Leukemia 17: 1738-1752, 2003, Nakao supra).

According to another embodiment the FLT3 mutation is a missense mutation at aspartic acid residue 835.

As used herein, the term "peptide" encompasses native peptides (either degradation products, synthetically synthesized peptides or recombinant peptides) and peptidomimetics (typically, synthetically synthesized peptides), as well as peptoids and semipeptoids which are peptide analogs, which may have, for example, modifications rendering the peptides more stable while in a body or more capable of penetrating into cells.

The CXCR4-antagonistic peptides of the present invention are interchangeably referred to as, 4F-benzoyl-TN14003 (SEQ ID NO: 1) analogs and derivatives and are structurally and functionally related to the peptides disclosed in patent applications WO 2002/020561 and WO 2004/020462, also known as "T-140 analogs", as detailed hereinbelow. Without being bound by theory it is suggested that peptides of the present invention induce growth arrest and/or death of myeloid leukemia cells.

As used herein a "CXCR4-antagonistic peptide" is a peptide which reduces CXCR-4 activation, by at least 10 %, as compared to same in the absence of the peptide antagonist. According to a specific embodiment the peptide antagonist is a competitive inhibitor. According to a specific embodiment the peptide antagonist is a non-competitive inhibitor.

As used herein, the term "peptide" encompasses native peptides (either degradation products, synthetically synthesized peptides or recombinant peptides) and peptidomimetics (typically, synthetically synthesized peptides), as well as peptoids and semipeptoids which are peptide analogs, which may have, for example, modifications rendering the peptides more stable while in a body or more capable of penetrating into cells.

According to a specific embodiment, the peptide is no more than 100 amino acids in length. According to a specific embodiment, the peptide is 5-100 amino acids in length. According to a specific embodiment, the peptide is 5-50 amino acids in length. According to a specific embodiment, the peptide is 5-20 amino acids in length. According to a specific embodiment, the peptide is 5-15 amino acids in length. According to a specific embodiment, the peptide is 10-20 amino acids in length. According to a specific embodiment, the peptide is 10-15 amino acids in length.

According to specific embodiments, the CXCR4-antagonistic peptides of the present invention are for example, 4F-benzoyl-TN 14003 (SEQ ID NO: 1) analogs and derivatives and are structurally and functionally related to the peptides disclosed in patent applications WO 2002/020561 and WO 2004/020462, also known as "T-140 analogs", as detailed hereinbelow.

In various particular embodiments, the T-140 analog or derivative has an amino acid sequence as set forth in the following formula (I) or a salt thereof: wherein:
A₁ is an arginine, lysine, ornithine, citrulline, alanine or glutamic acid residue or a N-α-substituted derivative of these amino acids, or A₁ is absent;
A₂ represents an arginine or glutamic acid residue if A₁ is present, or A₂ represents an arginine or glutamic acid residue or a N-α-substituted derivative of these amino acids if A₁ is absent;
A₃ represents an aromatic amino acid residue;
A₄, A₅ and A₉ each independently represents an arginine, lysine, ornithine, citrulline, alanine or glutamic acid residue;
A₆ represents a proline, glycine, ornithine, lysine, alanine, citrulline, arginine or glutamic acid residue;
A₇ represents a proline, glycine, ornithine, lysine, alanine, citrulline or arginine residue;
A₈ represents a tyrosine, phenylalanine, alanine, naphthylalanine, citrulline or glutamic acid residue;
A₁₀ represents a citrulline, glutamic acid, arginine or lysine residue;
A₁₁ represents an arginine, glutamic acid, lysine or citrulline residue wherein the C-terminal carboxyl may be derivatized;
and the cysteine residue of the 4-position or the 13-position can form a disulfide bond, and the amino acids can be of either L or D form.

Exemplary peptides according to formula (I) are peptides having an amino acid sequence as set forth in any one of SEQ ID NOS:1-72, as presented in Table 1 hereinbelow.

**Table 1 - T-140 and currently preferred T-140 analogs**

| Analog | SEQ ID NO: | Amino acid sequence |
|---|---|---|
| 4F-benzoyl-TN14003 | 1 | 4F-benzoyl-Arg-Arg-Nal-Cys-Tyr-Cit-Lys-DLys-Pro-Tyr-Arg-Cit-Cys-Arg-NH₂ |
| AcTC 14003 | 2 | Ac-Arg-Arg-Nal-Cys-Tyr-Cit-Lys-DLys-Pro-Tyr-Arg-Cit-Cys-Arg-OH |
| AcTC 14005 | 3 | Ac-Arg-Arg-Nal-Cys-Tyr-Arg-Lys-DCit-Pro-Tyr-Arg-Cit-Cys-Arg-OH |
| AcTC14011 | 4 | Ac-Arg-Arg-Nal-Cys-Tyr-Cit-Lys-DCit-Pro-Tyr-Arg-Cit-Cys-Arg-OH |
| AcTC14013 | 5 | Ac-Arg-Arg-Nal-Cys-Tyr-Cit-Lys-DLys-Pro-Tyr-Cit-Cit-Cys-Arg-OH |
| AcTC14015 | 6 | Ac-Cit-Arg-Nal-Cys-Tyr-Cit-Lys-DLys-Pro-Tyr-Arg-Cit-Cys-Arg-OH |
| AcTC 14017 | 7 | Ac-Cit-Arg-Nal-Cys-Tyr-Arg-Lys-DCit-Pro-Tyr-Arg-Cit-Cys-Arg-OH |
| AcTC14019 | 8 | Ac-Arg-Arg-Nal-Cys-Tyr-Arg-Lys-DCit-Pro-Tyr-Cit-Cit-Cys-Arg-OH |
| AcTC14021 | 9 | Ac-Cit-Arg-Nal-Cys-Tyr-Arg-Lys-DLys-Pro-Tyr-Cit-Cit-Cys-Arg-OH |
| AcTC14012 | 10 | Ac-Arg-Arg-Nal-Cys-Tyr-Cit-Lys-DCit-Pro-Tyr-Arg-Cit-Cys-Arg-NH₂ |
| AcTC14014 | 11 | Ac-Arg-Arg-Nal-Cys-Tyr-Cit-Lys-DLys-Pro-Tyr-Cit-Cit-Cys-Arg-NH₂ |
| AcTC14016 | 12 | Ac-Cit-Arg-Nal-Cys-Tyr-Cit-Lys-DLys-Pro-Tyr-Arg-Cit-Cys-Arg-NH₂ |
| AcTC14018 | 13 | Ac-Cit-Arg-Nal-Cys-Tyr-Arg-Lys-DCit-Pro-Tyr-Arg-Cit-Cys-Arg-NH₂ |
| AcTC 14020 | 14 | Ac-Arg-Arg-Nal-Cys-Tyr-Arg-Lys-DCit-Pro-Tyr-Cit-Cit-Cys-Arg-NH₂ |
| AcTC 14022 | 15 | Ac-Cit-Arg-Nal-Cys-Tyr-Arg-Lys-DLys-Pro-Tyr-Cit-Cit-Cys-Arg-NH₂ |
| TE14001 | 16 | H-DGlu-Arg-Nal-Cys-Tyr-Arg-Lys-DLys-Pro-Tyr-Arg-Cit-Cys-Arg-OH |
| TE14002 | 17 | H-Arg-Glu-Nal-Cys-Tyr-Arg-Lys-DLys-Pro-Tyr-Arg-Cit-Cys-Arg-OH |
| TE14003 | 18 | H-Arg-Arg-Nal-Cys-Tyr-Glu-Lys-DLys-Pro-Tyr-Arg-Cit-Cys-Arg-OH |
| TE14004 | 19 | H-Arg-Arg-Nal-Cys-Tyr-Arg-Glu-DLys-Pro-Tyr-Arg-Cit-Cys-Arg-OH |
| TE14005 | 20 | H-Arg-Arg-Nal-Cys-Tyr-Arg-Lys-DGlu-Pro-Tyr-Arg-Cit-Cys-Arg-OH |
| TE14006 | 21 | H-Arg-Arg-Nal-Cys-Tyr-Arg-Lys-DLys-Pro-Tyr-Glu-Cit-Cys-Arg-OH |
| TE14007 | 22 | H-Arg-Arg-Nal-Cys-Tyr-Arg-Lys-DLys-Pro-Tyr-Arg-Cit-Cys-Glu-OH |
| TE14011 | 23 | H-Arg-Arg-Nal-Cys-Tyr-Cit-Lys-DGlu-Pro-Tyr-Arg-Cit-Cys-Arg-NH₂ |
| TE14012 | 24 | H-Arg-Arg-Nal-Cys-Tyr-DGlu-Lys-DCit-Pro-Tyr-Arg-Cit-Cys-Arg-NH₂ |
| TE14013 | 25 | H-Arg-Arg-Nal-Cys-Tyr-DGlu-Lys-DGlu-Pro-Tyr-Arg-Cit-Cys-Arg-NH₂ |
| TE14014 | 26 | H-DGlu-Arg-Nal-Cys-Tyr-Cit-Lys-DGlu-Pro-Tyr-Arg-Cit-Cys-Arg-NH₂ |
| TE14015 | 27 | H-Arg-Arg-Nal-Cys-Tyr-Cit-Lys-DGlu-Pro-DGlu-Arg-Cit-Cys-Arg-NH₂ |
| TE14016 | 28 | H-Arg-Arg-Nal-Cys-Tyr-Cit-Lys-DGlu-Pro-Tyr-Arg-DGlu-Cys-Arg-NH2 |
| AcTE14014 | 29 | Ac-DGlu-Arg-Nal-Cys-Tyr-Cit-Lys-DGlu-Pro-Tyr-Arg-Cit-Cys-Arg-NH₂ |
| AcTE14015 | 30 | Ac-Arg-Arg-Nal-Cys-Tyr-Cit-Lys-DGlu-Pro-DGlu-Arg-Cit-Cys-Arg-NH₂ |
| AcTE14016 | 31 | Ac-Arg-Arg-Nal-Cys-Tyr-Cit-Lys-DGlu-Pro-Tyr-Arg-DGlu-Cys-Arg-NH₂ |
| TF1: AcTE14011 | 32 | Ac-Arg-Arg-Nal-Cys-Tyr-Cit-Lys-DGlu-Pro-Tyr-Arg-Cit-Cys-Arg-NH₂ |
| TF2: guanyl-TE14011 | 33 | guanyl-Arg-Arg-Nal-Cys-Tyr-Cit-Lys-DGlu-Pro-Tyr-Arg-Cit-Cys-Arg-NH₂ |
| TF3: TMguanyl-TE14011 | 34 | TMguanyl-Arg-Arg-Nal-Cys-Tyr-Cit-Lys-DGlu-Pro-Tyr-Arg-Cit-Cys-Arg-NH₂ |
| TF4: TMguanyl-TE14011 (2-14) | 35 | TMguanyl-Arg-Nal-Cys-Tyr-Cit-Lys-DGlu-Pro-Tyr-Arg-Cit-Cys-Arg-NH₂ |
| TF5: 4F-benzoyl-TE14011 | 36 | 4F-benzoyl-Arg-Arg-Nal-Cys-Tyr-Cit-Lys-DGlu-Pro-Tyr-Arg-Cit-Cys-Arg-NH₂ |
| TF6: 2F-benzoyl-TE14011 | 37 | 2F-benzoyl-Arg-Arg-Nal-Cys-Tyr-Cit-Lys-DGlu-Pro-Tyr-Arg-Cit-Cys-Arg-NH₂ |
| TF7: APA-TE14011 (2-14) | 38 | APA-Arg-Nal-Cys-Tyr-Cit-Lys-DGlu-Pro-Tyr-Arg-Cit-Cys-Arg-NH₂ |
| TF8: desamino-R-TE14011 (2-14) | 39 | desamino-R-Arg-Nal-Cys-Tyr-Cit-Lys-DGlu-Pro-Tyr-Arg-Cit-Cys-Arg-NH₂ |
| TF9: guanyl-TE14011 (2-14) | 40 | Guanyl-Arg-Nal-Cys-Tyr-Cit-Lys-DGlu-Pro-Tyr-Arg-Cit-Cys-Arg-NH₂ |
| TF10: succinyl-TE14011 (2-14) | 41 | succinyl-Arg-Nal-Cys-Tyr-Cit-Lys-DGlu-Pro-Tyr-Arg-Cit-Cys-Arg-NH₂ |
| TF11: glutaryl-TE14011 (2-14) | 42 | glutaryl-Arg-Nal-Cys-Tyr-Cit-Lys-DGlu-Pro-Tyr-Arg-Cit-Cys-Arg-NH₂ |
| TF12: deaminoTMG-APA-TE14011 (2-14) | 43 | deaminoTMG-APA-Arg-Nal-Cys-Tyr-Cit-Lys-DGlu-Pro-Tyr-Arg-Cit-Cys-Arg-NH₂ |
| TF15: H-Arg-CH2NH-RTE14011 (2-14) | 44 | R-CH2-Arg-Nal-Cys-Tyr-Cit-Lys-DGlu-Pro-Tyr-Arg-Cit-Cys-Arg-NH₂ |
| TF17: TE14011 (2-14) | 45 | H-Arg-Nal-Cys-Tyr-Cit-Lys-DGlu-Pro-Tyr-Arg-Cit-Cys-Arg-NH₂ |
| TF18: TMguanyl-TC14012 | 46 | TMguanyl-Arg-Arg-Nal-Cys-Tyr-Cit-Lys-DCit-Pro-Tyr-Arg-Cit-Cys-Arg-NH₂ |
| TF19: ACA-TC14012 | 47 | ACA-Arg-Arg-Nal-Cys-Tyr-Cit-Lys-DCit-Pro-Tyr-Arg-Cit-Cys-Arg-NH₂ |
| TF20: ACA-T140 | 48 | ACA-Arg-Arg-Nal-Cys-Tyr-Arg-Lys-DLys-Pro-Tyr-Arg-Cit-Cys-Arg-OH |
| TZ14011 | 49 | H-Arg-Arg-Nal-Cys-Tyr-Cit-Arg-DLys-Pro-Tyr-Arg-Cit-Cys-Arg-NH₂ |
| AcTZ14011 | 50 | Ac-Arg-Arg-Nal-Cys-Tyr-Cit-Arg-DLys-Pro-Tyr-Arg-Cit-Cys-Arg-NH₂ |
| AcTN14003 | 51 | Ac-Arg-Arg-Nal-Cys-Tyr-Cit-Lys-DLys-Pro-Tyr-Arg-Cit-Cys-Arg-NH₂ |
| AcTN14005 | 52 | Ac-Arg-Arg-Nal-Cys-Tyr-Arg-Lys-DCit-Pro-Tyr-Arg-Cit-Cys-Arg-NH₂ |
| 4F-benzoyl-TN14011-Me | 53 | 4F-benzoyl-Arg-Arg-Nal-Cys-Tyr-Cit-Lys-DGlu-Pro-Tyr-Arg-Cit-Cys-Arg-NHMe |
| 4F-benzoyl-TN14011-Et | 54 | 4F-benzoyl-Arg-Arg-Nal-Cys-Tyr-Cit-Lys-DGlu-Pro-Tyr-Arg-Cit-Cys-Arg-NHEt |
| 4F-benzoyl-TN14011-iPr | 55 | 4F-benzoyl-Arg-Arg-Nal-Cys-Tyr-Cit-Lys-DGlu-Pro-Tyr-Arg-Cit-Cys-Arg-NHiPr |
| 4F-benzoyl-TN14011-tyramine | 56 | 4F-benzoyl-Arg-Arg-Nal-Cys-Tyr-Cit-Lys-DGlu-Pro-Tyr-Arg-Cit-Cys-Arg-tyramine |
| TA14001 | 57 | H-Ala-Arg-Nal-Cys-Tyr-Arg-Lys-DLys-Pro-Tyr-Arg-Cit-Cys-Arg-OH |
| TA14005 | 58 | H-Arg-Arg-Nal-Cys-Tyr-Ala-Lys-DLys-Pro-Tyr-Arg-Cit-Cys-Arg-OH |
| TA14006 | 59 | H-Arg-Arg-Nal-Cys-Tyr-Arg-Ala-DLys-Pro-Tyr-Arg-Cit-Cys-Arg-OH |
| TA14007 | 60 | H-Arg-Arg-Nal-Cys-Tyr-Arg-Lys-DAla-Pro-Tyr-Arg-Cit-Cys-Arg-OH |
| TA14008 | 61 | H-Arg-Arg-Nal-Cys-Tyr-Arg-Lys-DLys-Ala-Tyr-Arg-Cit-Cys-Arg-OH |
| TA14009 | 62 | H-Arg-Arg-Nal-Cys-Tyr-Arg-Lys-DLys-Pro-Ala-Arg-Cit-Cys-Arg-OH |
| TA14010 | 63 | H-Arg-Arg-Nal-Cys-Tyr-Arg-Lys-DLys-Pro-Tyr-Ala-Cit-Cys-Arg-OH |
| TC14001 | 64 | H-Cit-Arg-Nal-Cys-Tyr-Arg-Lys-DLys-Pro-Tyr-Arg-Cit-Cys-Arg-OH |
| TC14003 | 65 | H-Arg-Arg-Nal-Cys-Tyr-Cit-Lys-DLys-Pro-Tyr-Arg-Cit-Cys-Arg-OH |
| TN14003 | 66 | H-Arg-Arg-Nal-Cys-Tyr-Cit-Lys-DLys-Pro-Tyr-Arg-Cit-Cys-Arg-NH₂ |
| TC14004 | 67 | H-Arg-Arg-Nal-Cys-Tyr-Arg-Cit-DLys-Pro-Tyr-Arg-Cit-Cys-Arg-OH |
| TC14012 | 68 | H-Arg-Arg-Nal-Cys-Tyr-Cit-Lys-DCit-Pro-Tyr-Arg-Cit-Cys-Arg-NH₂ |
| T-140 | 69 | H-Arg-Arg-Nal-Cys-Tyr-Arg-Lys-DLys-Pro-Tyr-Arg-Cit-Cys-Arg-OH |
| TC14011 | 70 | H-Arg-Arg-Nal-Cys-Tyr-Cit-Lys-DCit-Pro-Tyr-Arg-Cit-Cys-Arg-OH |
| TC14005 | 71 | H-Arg-Arg-Nal-Cys-Tyr-Arg-Lys-DCit-Pro-Tyr-Arg-Cit-Cys-Arg-OH |
| TC14018 | 72 | H-Cit-Arg-Nal-Cys-Tyr-Arg-Lys-DCit-Pro-Tyr-Arg-Cit-Cys-Arg-NH₂ |

According to a specific embodiment, in each one of SEQ ID NOS:1-72, two cysteine residues are coupled in a disulfide bond.

In another embodiment, the analog or derivative has an amino acid sequence as set forth in SEQ ID NO:65 (H-Arg-Arg-Nal-Cys-Tyr-Cit-Lys-DLys-Pro-Tyr-Arg-Cit-Cys-Arg-OH; TC14003).

In another embodiment, the peptide used in the compositions and methods of the invention consists essentially of an amino acid sequence as set forth in SEQ ID NO:1. In another embodiment, the peptide used in the compositions and methods of the invention comprises an amino acid sequence as set forth in SEQ ID NO:1. In another embodiment, the peptide is at least 60%, at least 70% or at least 80% homologous to SEQ ID NO:1. In another embodiment, the peptide is at least 90% homologous to SEQ ID NO:1. In another embodiment, the peptide is at least about 95% homologous to SEQ ID NO:1. Each possibility represents a separate embodiment of the present invention.

In various other embodiments, the peptide is selected from SEQ ID NOS:1-72, wherein each possibility represents a separate embodiment of the present invention.

In another embodiment, the peptide has an amino acid sequence as set forth in any one of SEQ ID NOS: 1-4, 10, 46, 47, 51-56, 65, 66, 68, 70 and 71. In another embodiment, the peptide has an amino acid sequence as set forth in any one of SEQ ID NOS: 4, 10, 46, 47, 68 and 70. In another embodiment, the peptide has an amino acid sequence as set forth in any one of SEQ ID NOS:1, 2, 51, 65 and 66. In another embodiment, the peptide has an amino acid sequence as set forth in any one of SEQ ID NOS:53-56.

In an embodiment, the peptide has an amino acid sequence as set forth in SEQ ID NO:1. In another embodiment, the peptide has an amino acid sequence as set forth in SEQ ID NO:2. In another embodiment, the peptide has an amino acid sequence as set forth in SEQ ID NO:51. In another embodiment, the peptide has an amino acid sequence as set forth in SEQ ID NO:66.

Other CXCR4 peptide inhibitors (antagonists) include but are not limited to LY2510924 (by Lilly Oncology), CTCE-9908 (Huang et al. 2009 Journal of Surgical Research 155:231-236), Fc131 analogs and nanobodies as specified in the citations below:
Tan NC, Yu P, Kwon Y-U, Kodadek T. High-throughput evaluation of relative cell permeability between peptoids and peptides. Bioorg Med Chem. 2008;16:5853-61.
Kwon Y-U, Kodadek T. Quantitative evaluation of the relative cell permeability of peptoids and peptides. J Am Chem Soc. 2007;129:1508.
Miller S, Simon R, Ng S, Zuckermann R, Kerr J, Moos W. Comparison of the proteolytic susceptibilities of homologous L-amino acid, D-amino acid, and N-substituted glycine peptide and peptoid oligomers. Drug Dev Res. 1995;35:20-32.
Yoshikawa Y, Kobayashi K, Oishi S, Fujii N, Furuya T. Molecular modeling study of cyclic pentapeptide CXCR4 antagonists: new insight into CXCR4-FC131 interactions. Bioorg Med Chem Lett. 2012;22:2146-50.
Jaähnichen S, Blanchetot C, Maussang D, Gonzalez-Pajuelo M, Chow KY, Bosch L, De Vrieze S, Serruys B, Ulrichts H, Vandevelde W. CXCR4 nanobodies (VHH-based single variable domains) potently inhibit chemotaxis and HIV-1 replication and mobilize stem cells. Proc Natl Acad Sci USA. 2010;107:20565-70.

The CXCR4-antagonistic peptide of the present invention is used for treating a subject having AML with a FLT3 mutation.

As used herein, the term "treating" refers to inhibiting, preventing or arresting the development of a pathology (disease, disorder or condition i.e., acute myeloid leukemia with a FLT3 mutation) and/or causing the reduction, remission, or regression of a pathology. Those of skill in the art will understand that various methodologies and assays can be used to assess the development of a pathology; and similarly, various methodologies and assays may be used to assess the reduction, remission or regression of a pathology.

As used herein, the term "preventing" refers to keeping a disease, disorder or condition from occurring in a subject who may be at risk for the disease, but has not yet been diagnosed as having the disease.

As used herein, the term "subject" includes mammals, preferably human beings at any age which suffer from the pathology.

The CXCR4-antagonistic peptide of the present invention can be administered to the subject either alone or in combination with one or more chemotherapeutic agents.

As used herein, the phrase "chemotherapeutic agent" refers to any chemical agent with therapeutic usefulness in the treatment of cancer. Chemotherapeutic agents as used herein encompass both chemical and biological agents. These agents function to inhibit a cellular activity upon which the cancer cell depends for continued survival. Categories of chemotherapeutic agents include alkylating/alkaloid agents, antimetabolites, hormones or hormone analogs, and miscellaneous antineoplastic drugs. Most if not all of these drugs are directly toxic to cancer cells and do not require immune stimulation. Suitable chemotherapeutic agents are described, for example, in Slapak and Kufe, Principles of Cancer Therapy, Chapter 86 in Harrison's Principles of Internal medicine, 14th edition; Perry et al., Chemotherapeutic, Ch 17 in Abeloff, Clinical Oncology 2nd ed., 2000 ChrchillLivingstone, Inc.; Baltzer L. and Berkery R. (eds): Oncology Pocket Guide to Chemotherapeutic, 2nd ed. St. Louis, mosby-Year Book, 1995; Fischer D. S., Knobf M. F., Durivage H.J. (eds): The Cancer Chemotherapeutic Handbook, 4th ed. St. Louis, Mosby-Year Handbook.

In some embodiments the chemotherapeutic agent of the present invention is cytarabine (cytosine arabinoside, Ara-C, Cytosar-U), quizartinib (AC220), sorafenib (BAY 43-9006), lestaurtinib (CEP-701), midostaurin (PKC412), carboplatin, carmustine, chlorambucil, dacarbazine, ifosfamide, lomustine, mechlorethamine, procarbazine, pentostatin, (2'deoxycoformycin), etoposide, teniposide, topotecan, vinblastine, vincristine, paclitaxel, dexamethasone, methylprednisolone, prednisone, all-trans retinoic acid, arsenic trioxide, interferon-alpha, rituximab (Rituxan®), gemtuzumab ozogamicin, imatinib mesylate, Cytosar-U), melphalan, busulfan (Myleran®), thiotepa, bleomycin, platinum (cisplatin), cyclophosphamide, Cytoxan®)., daunorubicin, doxorubicin, idarubicin, mitoxantrone, 5-azacytidine, cladribine, fludarabine, hydroxyurea, 6-mercaptopurine, methotrexate, 6-thioguanine, or any combination thereof.

In one embodiment the chemotherapeutic agent is cytarabine (ARA-C).

In another embodiment the chemotherapeutic agent is quizartinib (AC220).

Interestingly, the combination of the CXCR4 peptide antagonist (e.g., SEQ ID NO: 1) with chemotherapeutic agent (e.g., AC220) produces a synergy in the elicitation of apoptosis of AML cells.

The CXCR4-antagonistic peptide and the chemotherapeutic agent of the invention may be administered to the subject concomitantly or sequentially.

The CXCR4-antagonistic peptide of the invention can be administered to the subject as an active ingredient *per se,* or in a pharmaceutical composition where the active ingredient is mixed with suitable carriers or excipients.

As used herein a "pharmaceutical composition" refers to a preparation of one or more of the active ingredients described herein with other chemical components such as physiologically suitable carriers and excipients. The purpose of a pharmaceutical composition is to facilitate administration of a compound to an organism.

Herein the term "active ingredient" refers to the peptides accountable for the biological effect. Optionally, a plurality of active ingredient may be included in the formulation such as chemotherapeutic, radiation agents and the like, as further described hereinbelow.

Hereinafter, the phrases "physiologically acceptable carrier" and "pharmaceutically acceptable carrier", which may be used interchangeably, refer to a carrier or a diluent that does not cause significant irritation to an organism and does not abrogate the biological activity and properties of the administered compound.

Herein, the term "excipient" refers to an inert substance added to a pharmaceutical composition to further facilitate administration of an active ingredient. Examples, without limitation, of excipients include calcium carbonate, calcium phosphate, various sugars and types of starch, cellulose derivatives, gelatin, vegetable oils, and polyethylene glycols.

Techniques for formulation and administration of drugs may be found in the latest edition of "Remington's Pharmaceutical Sciences", Mack Publishing Co., Easton, PA, (Remington: The Science and Practice of Pharmacy, Gennaro, A., Lippincott, Williams & Wilkins, Philadelphia, Pa., 20th ed, 2000).

Pharmaceutical compositions of the present invention may be manufactured by processes well known in the art, e.g., by means of conventional mixing, dissolving, granulating, dragee-making, levigating, emulsifying, encapsulating, entrapping, or lyophilizing processes.

Pharmaceutical compositions for use in accordance with the present invention thus may be formulated in conventional manner using one or more physiologically acceptable carriers comprising excipients and auxiliaries, which facilitate processing of the active ingredients into preparations that can be used pharmaceutically. Proper formulation is dependent upon the route of administration chosen.

In one embodiment, the peptide of the invention or the pharmaceutical composition comprising same is administered subcutaneously.

In another embodiment, the peptide of the invention or the pharmaceutical composition comprising same is administered intravenously.

For injection, the active ingredients of the pharmaceutical composition may be formulated in aqueous solutions (e.g., WFI), preferably in physiologically compatible buffers such as Hank's solution, Ringer's solution, or physiological salt buffer.

Pharmaceutical compositions for potential administration include aqueous solutions of the active preparation in water-soluble form. Additionally, suspensions of the active ingredients may be prepared as appropriate oily or water-based injection suspensions. Suitable lipophilic solvents or vehicles include fatty oils such as sesame oil, or synthetic fatty acid esters such as ethyl oleate, triglycerides, or liposomes. Aqueous injection suspensions may contain substances that increase the viscosity of the suspension, such as sodium carboxymethyl cellulose, sorbitol, or dextran. Optionally, the suspension may also contain suitable stabilizers or agents that increase the solubility of the active ingredients, to allow for the preparation of highly concentrated solutions.

Alternatively, the active ingredient may be in powder form for constitution with a suitable vehicle, e.g., a sterile, pyrogen-free, water-based solution, before use.

Alternative embodiments include depots providing sustained release or prolonged duration of activity of the active ingredient in the subject, as are well known in the art.

Pharmaceutical compositions suitable for use in the context of the present invention include compositions wherein the active ingredients are contained in an amount effective to achieve the intended purpose. Determination of a therapeutically effective amount is well within the capability of those skilled in the art, especially in light of the detailed disclosure provided herein.

For any preparation used in the methods of the invention, the therapeutically effective amount or dose can be estimated initially from in vitro and cell culture assays. For example, a dose can be formulated in animal models to achieve a desired concentration or titer. Such information can be used to more accurately determine useful doses in humans.

Toxicity and therapeutic efficacy of the active ingredients described herein can be determined by standard pharmaceutical procedures in vitro, in cell cultures or experimental animals (see the Examples section which follows, and Sekido et al. 2002 Cancer Genet Cytogenet 137(1):33-42). The data obtained from these in vitro and cell culture assays and animal studies can be used in formulating a range of dosage for use in human. The dosage may vary depending upon the dosage form employed and the route of administration utilized. The exact formulation, route of administration and dosage can be chosen by the individual physician in view of the patient's condition. (See e.g., Fingl, et al., 1975, in "The Pharmacological Basis of Therapeutics", Ch. 1 p.1).

In some embodiments the daily dose of the CXCR4-antagonistic peptide of the invention or the pharmaceutical composition comprising same is ranging between 0.1 to 10 mg/kg of body weight, between 0.1 to 2 mg/kg of body weight, between 0.1 to 1 mg/kg of body weight, between 0.3 to 10 mg/kg of body weight, between 0.3 to 2 mg/kg of body weight, between 0.3 to 1 mg/kg of body weight or between 0.3 to 0.9 mg/kg of body weight.

In some embodiments the daily dose the chemotherapeutic agent of the invention or the pharmaceutical composition comprising same is ranging between 1 to 10 g per square meter of body area, between 1.5 to 5 g per square meter of body area or between 2 to 4 g per square meter of body area.

With respect to duration and frequency of treatment, it is typical for skilled clinicians to monitor subjects in order to determine when the treatment is providing therapeutic benefit, and to determine whether to increase or decrease dosage, increase or decrease administration frequency, discontinue treatment, resume treatment or make other alteration to treatment regimen. The dosing schedule can vary depending on a number of clinical factors, such as blood counts (e.g., red or white blood cell levels, hemoglobin level, etc.) the subject sensitivity to the peptide. The desired dose can be administered at one time or divided into sub-doses, e.g., 2-4 sub-doses and administered over a period of time, e.g., at appropriate intervals through the day or other appropriate schedule. Such sub-doses can be administered as unit dosage forms.

In some embodiments the CXCR4-antagonistic peptide of the invention is administered for a period of at least 1 day, at least 2 days, at least 3 days, at least 4 days, at least 5 days, at least 6 days, at least 1 week, at least 2 weeks, at least 3 weeks, at least 1 month, or at least 2 months prior to administering of the chemotherapeutic agent.

The active ingredients described herein i.e., CXCR4 antagonistic peptide and chemotherapeutic agent can be packaged in an article of manufacture. According to an embodiment of the invention such an article may comprise at least two separate containers (e.g., not more than 3 containers). One container packaging the CXCR-4 peptide antagonist (e.g., peptide set forth in SEQ ID NO: 1) and another container which packages the chemotherapy (e.g., ara-C). The article of manufacture may comprise a label and/or instructions for the treatment of myeloid leukemia (e.g., AML).

Alternatively or additionally, the CXCR4 inhibitor (e.g., SEQ ID NO: 1) and chemotherapy (cytarabine) can be formulated in a pharmaceutical composition as described above as a co-formulation.

Thus, compositions (CXCR4 antagonist, chemotherapy or a combination of same) and/or articles of some embodiments of the invention may, if desired, be presented in a pack or dispenser device, such as an FDA approved kit, which may contain one or more unit dosage forms containing the active ingredient. The pack may, for example, comprise metal or plastic foil, such as a blister pack. The pack or dispenser device may be accompanied by instructions for administration. The pack or dispenser may also be accommodated by a notice associated with the container in a form prescribed by a governmental agency regulating the manufacture, use or sale of pharmaceuticals, which notice is reflective of approval by the agency of the form of the compositions or human or veterinary administration. Such notice, for example, may be of labeling approved by the U.S. Food and Drug Administration for prescription drugs or of an approved product insert. Compositions comprising a preparation of the invention formulated in a compatible pharmaceutical carrier may also be prepared, placed in an appropriate container (e.g., lyophilized vial), and labeled for treatment of an indicated condition, as is further detailed above.

As used herein the term "about" refers to ± 10 %.

As used herein the term "method" refers to manners, means, techniques and procedures for accomplishing a given task including, but not limited to, those manners, means, techniques and procedures either known to, or readily developed from known manners, means, techniques and procedures by practitioners of the chemical, pharmacological, biological, biochemical and medical arts.

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable subcombination or as suitable in any other described embodiment of the invention. Certain features described in the context of various embodiments are not to be considered essential features of those embodiments, unless the embodiment is inoperative without those elements.

Various embodiments and aspects of the present invention as delineated hereinabove and as claimed in the claims section below find experimental support in the following examples.

### EXAMPLES

Reference is now made to the following examples, which together with the above descriptions, illustrate the invention in a non limiting fashion.

Generally, the nomenclature used herein and the laboratory procedures utilized in the present invention include molecular, biochemical, microbiological and recombinant DNA techniques. Such techniques are thoroughly explained in the literature. See, for example, "Molecular Cloning: A laboratory Manual" Sambrook et al., (1989); "Current Protocols in Molecular Biology" Volumes I-III Ausubel, R. M., Ed. (1994); Ausubel et al., "Current Protocols in Molecular Biology", John Wiley and Sons, Baltimore, Maryland (1989); Perbal, "A Practical Guide to Molecular Cloning", John Wiley & Sons, New York (1988); Watson et al., "Recombinant DNA", Scientific American Books, New York; Birren et al. (Eds.) "Genome Analysis: A Laboratory Manual Series", Vols. 1-4, Cold Spring Harbor Laboratory Press, New York (1998); methodologies as set forth in U.S. Pat. Nos. 4,666,828; 4,683,202; 4,801,531; 5,192,659 and 5,272,057; "Cell Biology: A Laboratory Handbook", Volumes I-III Cellis, J. E., Ed. (1994); "Culture of Animal Cells - A Manual of Basic Technique" by Freshney, Wiley-Liss, N. Y. (1994), Third Edition; "Current Protocols in Immunology" Volumes I-III Coligan J. E., Ed. (1994); Stites et al. (Eds.), "Basic and Clinical Immunology" (8th Edition), Appleton & Lange, Norwalk, CT (1994); Mishell and Shiigi (Eds.), "Selected Methods in Cellular Immunology", W. H. Freeman and Co., New York (1980); available immunoassays are extensively described in the patent and scientific literature, see, for example, U.S. Pat. Nos. 3,791,932; 3,839,153; 3,850,752; 3,850,578; 3,853,987; 3,867,517; 3,879,262; 3,901,654; 3,935,074; 3,984,533; 3,996,345; 4,034,074; 4,098,876; 4,879,219; 5,011,771 and 5,281,521; "Oligonucleotide Synthesis" Gait, M. J., Ed. (1984); "Nucleic Acid Hybridization" Hames, B. D., and Higgins S. J., Eds. (1985); "Transcription and Translation" Hames, B. D., and Higgins S. J., Eds. (1984); "Animal Cell Culture" Freshney, R. I., Ed. (1986); "Immobilized Cells and Enzymes" IRL Press, (1986); "A Practical Guide to Molecular Cloning" Perbal, B., (1984) and "Methods in Enzymology" Vol. 1-317, Academic Press; "PCR Protocols: A Guide To Methods And Applications", Academic Press, San Diego, CA (1990); Marshak et al., "Strategies for Protein Purification and Characterization - A Laboratory Course Manual" CSHL Press (1996). Other general references are provided throughout this document. The procedures therein are believed to be well known in the art and are provided for the convenience of the reader.

### EXAMPLE 1

### The Effect of the CXCR4 Antagonistic Peptide BL-8040, Either Alone or in Combination with Chemotherapeutic Agents, on the Survival of AML Cells in vitro Materials and Methods

### Agents

BL-8040 (4F-benzoyl-TN14003; SEQ ID NO: 1) was synthesized and lyophilized by MSD N.V.

ARA-C (Cytarabine) was purchased from Hadassah cytotoxica pharmacy (Israel).

AC220 (Quizartinib) was purchased from Selleck chemicals, USA.

### AML Cells

The following cell lines were obtained from ATCC: MV4-11 (human AML cells with FLT3-ITD mutation) and HL60 (human AML cells with wild-type FLT3; FLT3-WT).

Human primary AML cells with FLT3-ITD mutation and with FLT3-WT were obtained from AML patients after getting their consent in accordance with regulations of Chaim Sheba Medical Center (Tel-Aviv, Israel). Peripheral blood mononuclear cells (PBMCs) were separated from blood samples by density-gradient centrifugation on Ficoll-Paque (Pharmacia Biotech, Uppsala, Sweden). The cells were suspended in 1 % fetal calf serum (FCS; Biological Industries, Kibbutz Beit Haemek, Israel), supplement with 10% DMSO then stored in liquid nitrogen. Prior to commencing a toxicity assay the isolated cells were thawed, re-suspended in Roswell Park Memorial Institute medium (RPMI 1640; Gibco BRL life technologies) supplemented with 20% FCS and incubated for 4 hr at 37°C. Isolated cells with FLT3-ITD mutation or with FLT3 wild type (FLT3-WT) were identified by using a procedure essentially as described by Levis and Small (Leukemia 17: 1738-1752, 2003).

### Survival assay procedure

Cells were seeded at 2x10⁵ cells / 250 µl per well into a 96-well plate in RPMI medium supplemented with 1% FCS with or without BL-8040 (8µM or 20µM), ARA-C (50 ng/ml) and AC220 (0.5 or 50 nM) or their combination. The cultures were incubated at 37°C in a humidified atmosphere containing 5% CO₂ for 48 hr. Following incubation the cells were stained with propidium iodide (PI; Sigma, St. Louis, MO; 1:1000) and the incidence of dead cells (% PI-positive) and the density of viable cells (PI-negative) were determined by FACScalibur using the procedure described by Beider and Begin (Exp Hematol 39: 282-92, 2011).

### Results

Exposure of human AML cells with wild-type FLT3 gene to BL-8040 resulted in an increase of dead cells incidence (% dead cells) and in a decrease of viable cells density (number of viable cells). However, most unexpectedly, the effect of BL-8040 alone on similar AML cells but with FLT3-ITD mutation (as opposed to wild-type FLT3) was substantially stronger, with higher levels of the percent of dead cells and lower levels in the number of viable cells. This differential effect of BL-8040 (i.e., SEQ

ID NO: 1, more effective against AML cells having FLT3-ITD mutation) was most surprising since AML with FLT3-ITD mutation is known to be refractory to standard chemotherapy.

FIG. 1A shows that exposure to BL-8040 (8µM) increased the percentage of dead human primary AML cells with FLT3-ITD by **79.3** %. In comparison, BL-8040 increased the percentage of dead human primary AML cells with wild-type FLT3 by only **13.7 %** (FIG. 2A).

FIG. 1B shows that exposure to BL-8040 (8µM) decreased the number of viable human primary AML-cells with FLT3-ITD by **28.8** %. In comparison, BL-8040 decreased the number of viable human primary AML cells with wild type FLT3 by only **16.1 %** (FIG. 2B).

FIG. 7A shows that exposure to BL-8040 (20 µM) increased the percentage of dead human primary AML cells with FLT3-ITD by **116.6** %. In comparison, BL-8040 increased the percentage of dead human primary AML cells with wild-type FLT3 by only **56.3** % (FIG. 8A).

FIG. 7B shows that BL-8040 (20 µM) decreased the number of viable human primary AML cells with FLT3-ITD by **50.0** %. In comparison, BL-8040 decreased the number of viable human primary AML cells with-wild type FLT3 by only **34.4** % (FIG. 8B).

When BL-8040 was combined with a chemotherapeutic agent (ARA-C or AC220) the combined effect of the mixture on the survival of AML cells (determined by the incidence of dead cells and by the density of remaining viable cells) was substantially stronger against AML cells with FLT3-ITD mutation than it was against AML cells with wild-type FLT3.

FIG. 1A shows that BL-8040 (8µM) combined with ARA-C (50 ng/ml) increased the percentage of dead human primary AML cells with FLT3-ITD by **110.3** %. In comparison, the same combination treatment increased the percentage of dead human primary AML cells with wild-type FLT3 by only **13.7** % (FIG. 2A).

FIG. 1B shows that BL-8040 (8µM) combined with ARA-C (50 ng/ml) decreased the number of viable human primary AML-cells with FLT3-ITD by **44.4** %. In comparison, the same combination treatment decreased the number of viable human primary AML cells with wild type FLT3 by only **3.3** % (FIG. 2B).

FIG. 3A shows that BL-8040 (20 µM) combined with ARA-C (50 ng/ml) increased the percentage of dead human MV4-11 AML cells with FLT3-ITD by **143.7** %. In comparison, the same combination treatment increased the percentage of dead human HL-60 AML cells with wild-type FLT3 by only **32.4** % (FIG. 4A).

FIG. 3B shows that BL-8040 (20 µM) combined with ARA-C (50 ng/ml) decreased the number of viable human MV4-11 AML cells with FLT3-ITD by **73.8** %. In comparison, the same combination treatment decreased the number of viable human HL-60 AML cells with wild type FLT3 by only **50.0** % (FIG. 4B).

FIG. 5A shows that BL-8040 (20 µM) combined with AC220 (0.5 µM) increased the percentage of dead human MV4-11 AML cells with FLT3-ITD by **218.2** %. In comparison, the same combination treatment increased the percentage of dead human HL-60 AML cells with wild-type FLT3 by only **8.8** % (FIG. 6A).

FIG. 5B shows that BL-8040 (20 µM) combined with AC220 (0.5 µM) decreased the number of viable human MV4-11 AML cells with FLT3-ITD by **78.8** %. In comparison, the same combination treatment decreased the number of viable human HL-60 AML cells with wild type FLT3 by only **51.4** % (FIG. 6B).

FIG. 7A shows that BL-8040 (20 µM) combined with AC220 (50 µM) increased the percentage of dead human MV4-11 AML cells with FLT3-ITD by **150.0** %. In comparison, the same combination treatment increased the percentage of dead human HL-60 AML cells with wild-type FLT3 by only **64.6** % (FIG. 8A).

FIG. 7B shows that BL-8040 (20 µM) combined with AC220 (50 µM) decreased the number of viable human MV4-11 AML cells with FLT3-ITD by **85.7** %. In comparison, the same combination treatment decreased the number of viable human HL-60 AML cells with wild type FLT3 by only **34.8** % (FIG. 8B).

These results clearly indicate that the CXCR4-antagonistic peptide BL-8040, either alone or in combination with chemotherapeutic agents, is uniquely advantageous for treating AML with FLT3-ITD mutation.

### EXAMPLE 2

### BL-8040 elicits apoptosis of AML cells in AML FLT3-ITD model which is further increased in the presence of AC220

The present inventors have studies the effect of BL-8040 on survival and apoptosis of AML cells with FLT3 mutation alone or in combined with the FLT3 inhibitor AC220.

**Methods:** The human AML MV4-11 cells (*FLT3-ITD*) was used. Cells were in-vitro incubated for 48 hrs in the presence of BL-8040 (20µM), AC220 (50nM) or their combination. The level of viable cells, percentage of apoptosis was evaluated by FACS.

In the in-vivo study an AML model of NOD SCID gamma (NSG) mice engrafted with MV4-11 cells was used. Three weeks after engraftment mice were treated daily for seven consecutive days with subcutaneous (SC) injection of BL-8040 (400 ug/mouse) or with oral administration of AC220 (10mg/Kg) or their combination. The survival and apoptosis of AML cells were examined in the blood, BM and spleen of engrafted mice.

The outline of the study is provided below.

**MIce**

| | |
|---|---|
| Control | 4 |
| BL8040 | 5 |
| AC220 | 5 |
| BL8040+AC220 | 5 |

Female mice at the age of 7-9 weeks
**day (-1)** 26/1/14: mice were irradiated with 200 rad 24 hr before cells injection **day 0** -27/1/14: mice were IV injected with 10x10⁶ of MV4-11 cells in total volume of 200 ul PBS.
**Day 16** - 12/2/14
   The level of hCD45+ cells in the blood was evaluated by FACS:
   50 ul of blood were lysed with 1 ml of ACK and incubated with APC- anti-human CD45 (1:40)
   cells were resuspended with 300 ul of PBS and read by FACS for 30 min at high speed following PI staining.
**day 17**-13/2/14- mice were SC injected with 400 ug/mouse of BKT140 (400-500-110)
   or with orally with AC220 or their combination
   13/2/14- 19/2/14 daily treatment of BKT140 and AC220 for 7 consecutive days
**Day 24** - 20/2/14 mice were sacrificed spleen, blood and 4 bones were taken from each mouse

### Results:

In-vitro, treatment of AML cells with BL-8040 directly inhibited cell growth by 35% and increased cell death by 40%. AC220 was found to induce cell death in 60% of the cells and the combination of BL-8040 with AC220 further increased the apoptotic effect of these agents achieving a 97% reduction in cell viability and inducing cell death by 93% of AML cells.

In-vivo, BL-8040 was found to reduce the percentage of alive AML blasts in the blood from 13.5% in the control to 1.7% (Figure 9). Treatment with AC220 with or without BL-8040 reduces this level to 0.1% (Figure 9). Interestingly, the level of total mouse WBC following AC220 was significantly reduced in 65% compared to the control (Figure 10). This deep reduction in normal WBC was prevented when AC220 was combined with BL-8040. BL-8040 was found to decrease the number of AML cell in the BM to 2.6% compared to 12.6% in the control mice while AC220 reduced this level to 0.05%. The combination of AC220 with BL-8040 was found to decrease this level to only 0.006% of AML cells in the BM with 3/5 mice with no AML cells at all in the BM (Figures 11A-B). Similar effect was observed in the spleen when BL-8040 reduced the level of AML cells from 21% in the control to 0.4% and AC220 reduced this level to 0.09%. The combination of AC220 with BL-8040 was further decreasing this level to 0.02% (Figures 12A-B). The reduction in the number of AML cells in the blood, BM and spleen was accompanied with the induction of AML cells apoptosis (Figures 13A-B).

**Conclusions:** The CXCR4 antagonist BL-8040 was found to rapidly and efficiently induces cell death of AML cells both in-vitro and in-vivo and synergized with AC220. The combination of BL-8040 and AC220 was found to reduce minimal residual disease of AML cells. These results suggest potential therapeutic advantages of BL-8040 in FLT3-positive AML patients by targeting not only AML anchorage in the BM but AML survival as well. Furthermore, it could provide a rational basis for BL-8040 therapy in combination with the FLT3 inhibitor AC220.

## Claims

1. A CXCR4-antagonistic peptide for use in the treatment of AML with a FMS-like tyrosine kinase 3 (FLT3) mutation in a subject in need thereof, wherein said CXCR4-antagonistic peptide is as set forth in SEQ ID NO: 1.

2. The CXCR4-antagonistic peptide for use in the treatment of AML with a FMS-like tyrosine kinase 3 (FLT3) of claim 1, wherein said use further comprises a chemotherapeutic agent.

3. An article of manufacture for use in the treatment of AML with a FMS-like tyrosine kinase 3 (FLT3) mutation comprising a CXCR4-antagonistic peptide and a chemotherapeutic agent, wherein said CXCR4-antagonistic peptide is as set forth in SEQ ID NO: 1.

4. The article of manufacture for use of claim 3, wherein said CXCR4-antagonistic peptide and said chemotherapeutic agent are in separate containers.

5. The CXCR4-antagonistic peptide for use of claim 2 or article of manufacture for use of claim 3 or 4, wherein said chemotherapeutic agent is selected from cytarabine (cytosine arabinoside, Ara-C), quizartinib (AC220), sorafenib (BAY 43-9006), lestaurtinib (CEP-701), midostaurin (PKC412), carboplatin,carmustine, chlorambucil, dacarbazine, ifosfamide, lomustine, mechlorethamine, procarbazine, pentostatin (2'deoxycoformycin), etoposide, teniposide, topotecan, vinblastine, vincristine, paclitaxel, dexamethasone, methylprednisolone, prednisone, alltransretinoic acid, arsenic trioxide, interferon-alpha, rituximab, gemtuzumab ozogamicin, imatinib mesylate, melphalan, busulfan, thiotepa, bleomycin, platinum (cisplatin), cyclophosphamide, daunoirubicin, doxorubicin, idarubicin, mitoxantrone, 5-azacytidine, cladribine,fludarabine, hydroxyurea, 6-mercaptopurine, methotrexate, 6-thioguanine, or any combinatino thereof.

6. The CXCR4-antagonistic peptide for use of claim 2 or article of manufacture for use of claims 3 or 4, wherein said chemotherapeutic agent is quizartinib (AC220).

7. The CXCR4-antagonistic peptide for use of claim 2 or article of manufacture for use of claims 3-4 wherein said chemotherapeutic agent is cytarabine (ARA-C).

8. The CXCR4-antagonistic peptide for use of claim 2 or article of manufacture for use of any one of claims 3-5, wherein said chemotherapeutic agent synergizes with said CXCR4-antagonistic peptide in inducing apoptosis of AML cells.

9. The CXCR4-antagonistic peptide for use or article of manufacture for use of any one of claims 1-8, wherein said AML is minimal residual disease of AML.

10. The CXCR4-antagonistic peptide for use or article of manufacture for use of any one of claims 1-9, wherein said FLT3 mutation is a FLT3 internal tandem duplication (ITD) mutation.

11. The CXCR4-antagonistic peptide for use of any one of claims 1, 2 and 5-10, wherein said CXCR4-antagonistic peptide is for administration to said subject in a daily amount between 0.1 to 10 mg per kg of body weight.

12. The CXCR4-antagonistic peptide for use of any one of claims 1, 2 and 5-11, wherein said CXCR4-antagonistic peptide is for subcutaneous administration.

13. The CXCR4-antagonistic peptide for use of any one of claims 1, 2 and 5-11, wherein said CXCR4-antagonistic peptide is for intravenous administration.

## Patentansprüche

1. CXCR4-antagonistisches Peptid zur Verwendung bei der Behandlung von AML mit einer Mutation der FMS-ähnlichen Tyrosinkinase 3 (FLT3) in einem Individuum, das dies benötigt, wobei das CXCR4-antagonistische Peptid wie in SEQ ID Nr.: 1 angegeben ist.

2. CXCR4-antagonistisches Peptid zur Verwendung bei der Behandlung von AML mit einer FMS-ähnlichen Tyrosinkinase 3 (FLT3) nach Anspruch 1, wobei die Verwendung ferner ein chemotherapeutisches Mittel umfasst.

3. Erzeugnis zur Verwendung bei der Behandlung von AML mit einer Mutation der FMS-ähnlichen Tyrosinkinase 3 (FLT3), umfassend ein CXCR4-antagonistisches Peptid und ein chemotherapeutisches Mittel, wobei das CXCR4-antagonistische Peptid wie in SEQ ID Nr.: 1 angegeben ist.

4. Erzeugnis zur Verwendung nach Anspruch 3, wobei sich das CXCR4-antagonistische Peptid und das chemotherapeutische Mittel in separaten Behältern befinden.

5. CXCR4-antagonistisches Peptid zur Verwendung nach Anspruch 2 oder Erzeugnis zur Verwendung nach Anspruch 3 oder 4, wobei das chemotherapeutische Mittel ausgewählt ist aus Cytarabin (Cytosinarabinosid, Ara-C), Quizartinib (AC220), Sorafenib (BAY 43-9006), Lestaurtinib (CEP-701), Midostaurin (PKC412), Carboplatin, Carmustin, Chlorambucil, Dacarbazin, Ifosfamid, Lomustin, Mechlorethamin, Procarbazin, Pentostatin (2'-Deoxycoformycin), Etoposid, Teniposid, Topotecan, Vinblastin, Vincristin, Paclitaxel, Dexamethason, Methylprednisolon, Prednison, all-trans-Retinsäure, Arsentrioxid, Interferon-Alpha, Rituximab, Gemtuzumab-Ozogamicin, Imatinib-Mesylat, Melphalan, Busulfan, Thiotepa, Bleomycin, Platin (Cisplatin), Cyclophosphamid, Daunoirubicin, Doxorubicin, Idarubicin, Mitoxantron, 5-Azacytidin, Cladribin, Fludarabin, Hydroxyharnstoff, 6-Mercaptopurin, Methotrexat, 6-Thioguanin oder jeder Kombination davon.

6. CXCR4-antagonistisches Peptid zur Verwendung nach Anspruch 2 oder Erzeugnis zur Verwendung nach den Ansprüche 3 oder 4, wobei das chemotherapeutische Mittel Quizartinib (AC220) ist.

7. CXCR4-antagonistisches Peptid zur Verwendung nach Anspruch 2 oder Erzeugnis zur Verwendung nach den Ansprüchen 3 bis 4, wobei das chemotherapeutische Mittel Cytarabin (ARA-C) ist.

8. CXCR4-antagonistisches Peptid zur Verwendung nach Anspruch 2 oder Erzeugnis zur Verwendung nach einem der Ansprüche 3 bis 5, wobei das chemotherapeutische Mittel bei der Induktion der Apoptose von AML-Zellen eine Synergie mit dem CXCR4-antagonistischen Peptid aufweist.

9. CXCR4-antagonistisches Peptid zur Verwendung oder Erzeugnis zur Verwendung nach einem der Ansprüche 1 bis 8, wobei die AML eine minimale Resterkrankung von AML ist.

10. CXCR4-antagonistisches Peptid zur Verwendung oder Erzeugnis zur Verwendung nach einem der Ansprüche 1 bis 9, wobei die FLT3-Mutation eine interne Tandemduplikationsmutation (ITD) ist.

11. CXCR4-antagonistisches Peptid zur Verwendung nach einem der Ansprüche 1, 2 und 5 bis 10, wobei das CXCR4-antagonistische Peptid zur Verabreichung an das Individuum in einer täglichen Menge zwischen 0,1 und 10 mg pro kg Körpergewicht bestimmt ist.

12. CXCR4-antagonistisches Peptid zur Verwendung nach einem der Ansprüche 1, 2 und 5 bis 11, wobei das CXCR4-antagonistische Peptid zur subkutanen Verabreichung bestimmt ist.

13. CXCR4-antagonistisches Peptid zur Verwendung nach einem der Ansprüche 1, 2 und 5 bis 11, wobei das CXCR4-antagonistische Peptid zur intravenösen Verabreichung bestimmt ist.

## Revendications

1. Peptide antagoniste de CXCR4 pour son utilisation dans le traitement de la LMA avec une mutation de la tyrosine kinase 3 de type FMS (FLT3) chez un sujet le nécessitant, dans lequel ledit peptide antagoniste de CXCR4 est tel que présenté dans SEQ ID NO : 1.

2. Peptide antagoniste de CXCR4 pour son utilisation dans le traitement de la LMA avec une mutation de la tyrosine kinase 3 de type FMS (FLT3) selon la revendication 1, dans lequel ladite utilisation comprend en outre un agent chimiothérapeutique.

3. Article de fabrication pour son utilisation dans le traitement de la LMA avec une mutation de la tyrosine kinase 3 de type FMS (FLT3) comprenant un peptide antagoniste de CXCR4 et un agent chimiothérapeutique, dans lequel ledit peptide antagoniste de CXCR4 est tel que présenté dans SEQ ID NO : 1.

4. Article de fabrication pour son utilisation selon la revendication 3, dans lequel ledit peptide antagoniste de CXCR4 et ledit agent chimiothérapeutique sont dans des récipients séparés.

5. Peptide antagoniste de CXCR4 pour son utilisation selon la revendication 2 ou article de fabrication pour son utilisation selon la revendication 3 ou 4, dans lequel ledit agent chimiothérapeutique est sélectionné parmi la cytarabine (cytosine arabinoside, Ara-C), le quizartinib (AC220), le sorafénib (BAY 43-9006), le lestaurtinib (CEP-701), la midostaurine (PCK412), le carboplatine, la carmustine, le chlorambucil, la dacarbazine, l'ifosfamide, la lomustine, la méchloréthamine, la procarbazine, la pentostatine, la (2'désoxycoformycine), l'étoposide, le téniposide, le topotécan, la vinblastine, la vincristine, le paclitaxel, la déxamethasone, la méthylprednisolone, le prednisone, l'acide tout-trans rétinoïque, le trioxyde d'arsénique, l'interféron α, le rituximab, le gemtuzumab ozogamicine, le mésylate d'imatinib, le melphalan, le busulfan, le thiotépa, la bléomycine, le platine (cisplatine), le cyclophosphamide, la daunorubicine, la doxorubicine, l'idarubicine, la mitoxantrone, la 5-azacytidine, la cladribine, la fludarabine, l'hydroxyurée, la 6-mercaptopurine, le méthotrexate, la 6-thioguanine ou toute combinaison de ceux-ci.

6. Peptide antagoniste de CXCR4 pour son utilisation selon la revendication 2 ou article de fabrication pour son utilisation selon les revendications 3 ou 4, dans lequel ledit agent chimiothérapeutique est le quizartinib (AC220).

7. Peptide antagoniste de CXCR4 pour son utilisation selon la revendication 2 ou article de fabrication pour son utilisation selon les revendications 3 et 4 dans lequel ledit agent chimiothérapeutique est la cytarabine (ARA-C).

8. Peptide antagoniste de CXCR4 pour son utilisation selon la revendication 2 ou article de fabrication pour son utilisation selon l'une quelconque des revendications 3 à 5, dans lequel ledit agent chimiothérapeutique est synergique avec ledit peptide antagoniste de CXCR-4 dans l'induction de l'apoptose des cellules de LMA.

9. Peptide antagoniste de CXCR4 pour son utilisation ou article de fabrication pour son utilisation selon l'une quelconque des revendications 1 à 8, dans lequel ladite LMA est une maladie résiduelle minime de la LMA.

10. Peptide antagoniste de CXCR4 pour son utilisation ou article de fabrication pour son utilisation selon l'une quelconque des revendications 1 à 9, dans lequel ladite mutation de FLT3 est une mutation de duplication en tandem interne (ITD) de FTL3.

11. Peptide antagoniste de CXCR4 pour son utilisation selon l'une quelconque des revendications 1, 2 et 5 à 10, dans lequel ledit peptide antagoniste de CXCR4 est destiné à être administré au dit sujet en une quantité quotidienne comprise entre 0,1 et 10 mg par kg de poids corporel.

12. Peptide antagoniste de CXCR4 pour son utilisation selon l'une quelconque des revendications 1, 2 et 5 à 11, dans lequel ledit peptide antagoniste de CXCR4 est destiné à une administration sous-cutanée.

13. Peptide antagoniste de CXCR4 pour son utilisation selon l'une quelconque des revendications 1, 2 et 5 à 11, dans lequel ledit peptide antagoniste de CXCR4 est destiné à une administration intraveineuse.
